# EUROPEAN PATENT APPLICATION

(11) **EP 4 527 853 A1**
(43) Date of publication of application: **26.03.2025**
(21) Application number: 23807931.3
(22) Date of filing: 18.05.2023
(51) Int. Cl.: C07K 16/28, A61P 35/00, A61K 39/00

(54) **BISPECIFIC HUMANIZED SINGLE DOMAIN ANTIBODY TO PD-L1 AND CD47, AND USE THEREOF**

(30) Priority: 19.05.2022 KR 20220061242; 07.09.2022 KR 20220113486
(71) Applicant: Shaperon Inc., Seoul 06373 (KR); Seoul National University R & DB Foundation, Seoul 08826 (KR)
(72) Inventor: SEONG, Seung Yong, Seoul 05507 (KR); LEE, Sang Beum, Seoul 06374 (KR); KIM, Jeong Hwan, Seoul 07013 (KR)
(74) Representative: Vossius & Partner Patentanwälte Rechtsanwälte mbB
(86) International application number: PCT/KR2023/006794
(87) International publication number: WO 2023/224412

(57) **Abstract**

The present invention relates to a bispecific humanized single domain antibody targeting both PD-L1 and CD47, as well as uses thereof. Specifically, a humanized single domain antibody that binds bispecifically to PD-L1 and CD47, which are immune checkpoint proteins, has been developed, and its efficacy has been confirmed both *in vitro* and *in vivo.* Therefore, the bispecific humanized single domain antibody can be effectively used as an immune checkpoint inhibitor for immuno-oncology therapy.

## Description

### [Technical Field]

The present invention relates to a bispecific humanized single domain antibody targeting the immune checkpoint proteins PD-L1 and CD47, and uses thereof.

### [Background Art]

Recently, as the therapeutic efficacy of newly developed immuno-oncology therapies utilizing the human immune system has been demonstrated, traditional cancer treatment methods using chemotherapeutic agents and targeted therapeutics are being shifted toward immuno-oncology therapies employing immunotherapeutics.

In general, immune cells in cancer patients develop tolerance to cancer antigens, recognizing cancer cells yet remaining functionally suppressed, thereby failing to effectively eliminate cancer cells. The core of immunotherapy is to awaken immune cells in a state of tolerance and activate them to induce the destruction of cancer cells. Types of immunotherapy include cytokine therapies such as IFN-γ and IL-2, dendritic cell-based cancer vaccines, T cell-based cell therapies, and immune checkpoint inhibitors (ICIs) that block immune checkpoint proteins. These therapies are commonly referred to as immuno-oncology therapies. Among them, the immuno-oncology therapies being competitively developed by international pharmaceutical companies are immune checkpoint inhibitors.

Immune checkpoint proteins are cell membrane proteins that suppress the differentiation, proliferation, and activation of immune cells. Specifically, these proteins are generally expressed on activated T cells, where they reduce T cell proliferation, cytokine secretion, and cytotoxicity, and suppress excessive T cell activation, thereby being referred to as co-inhibitory molecules. Notably, T cells express co-inhibitory receptors such as CTLA-4 and PD-1, which regulate T cell activation through binding with their respective ligands, B7.1/2 and PD-L1. Meanwhile, PD-L1, which is expressed on cancer cells, plays a critical role as a molecular shield, inactivating tumor-specific T cells and inducing apoptosis, thereby protecting cancer cells from T cell-mediated immune attacks and contributing to the cancer immune evasion mechanism. Additionally, it has been reported that cancer patients with ectopic expression of PD-L1 on cancer cells have poorer prognoses compared to those without such expression.

Immune checkpoint inhibitors are agents that block the activity of immune checkpoint proteins involved in T cell suppression, thereby activating T cells to attack cancer cells. Representative antibodies used in this context include those targeting CTLA-4, PD-1, and PD-L1. The CTLA-4 inhibitor ipilimumab (Yervoy) was the first immune checkpoint inhibitor to receive FDA approval in 2011 as a second-line therapy for metastatic melanoma. Subsequently, in 2014, the PD-1 inhibitors nivolumab (Opdivo) and pembrolizumab (Keytruda) were approved by the FDA for metastatic melanoma. Later, in 2016, the PD-L1 inhibitor atezolizumab (Tecentriq) was approved for bladder cancer, while avelumab (Bavencio) was approved in 2017 for metastatic Merkel cell carcinoma, a type of skin cancer, and durvalumab (Imfinzi) for bladder cancer. In 2018, the PD-1 inhibitor cemiplimab (Libtayo) received FDA approval for cutaneous squamous cell carcinoma. Currently, these drugs are expanding their therapeutic indications, gaining FDA approvals for an increasing range of cancer types. As of 2019, six PD-1/PD-L1 inhibitors have received FDA approval for a total of 18 cancer types. Additionally, other immune modulatory proteins, such as B7-H4, ICOS, HVEM, PDL-2, and PVRIG, are entering preclinical trials as novel targets. Most of these therapies target molecules expressed on T cells.

To overcome the T cell-centric bias in target discovery, recent efforts have focused on developing inhibitors targeting immune checkpoint proteins expressed on myeloid lineage cells, such as macrophages and dendritic cells. Among these, CSF1R, CD47, and TLR7 are emerging as important targets.

PD-L1 (Programmed Death-Ligand 1), an immune checkpoint protein that enables tumor cells to evade immune system attacks by suppressing T cell activity, is primarily expressed on leukocytes in lymphoid and non-lymphoid tissues as well as on nonhematopoietic cells. Additionally, PD-L1 is expressed on the surface of various tumor cells, including those of colorectal cancer, pancreatic cancer, melanoma, and cervical cancer. In particular, PD-L1 interacts with PD-1 (Programmed Death-1), which is expressed on activated T cells, to negatively regulate T cell immune responses by inhibiting TCR-mediated T cell activation, cytokine release, and T cell proliferation.

CD47 (Cluster of Differentiation 47), which was first identified as a tumor antigen in human ovarian cancer in 1980, is expressed in various human tumor cells, including non-Hodgkin lymphoma (NHL), multiple myeloma (MM), acute lymphoblastic leukemia (ALL), acute myeloid leukemia (AML), chronic myeloid leukemia, bladder cancer, and solid tumors.

CD47 is expressed on the cell surface and interacts with SIRPalpha (Signal Regulatory Protein Alpha), thrombospondin-1 (TSP1), and integrin proteins, playing roles in cell apoptosis, proliferation, and immune response. Specifically, CD47 expressed on tumor cells interacts with SIRPα, which is expressed on the surface of macrophages, sending a "don't eat me" signal that allows tumor cells to evade phagocytosis by macrophages. Additionally, within the tumor microenvironment, CD47 inhibits angiogenesis and the functions of effector T cells, thereby promoting the proliferation and growth of tumor cells.

Magrolimab, developed by the global pharmaceutical company Gilead, is an antibody therapy targeting CD47 that is currently undergoing clinical trials. Recently, multinational global pharmaceutical companies have been actively engaged in the development of bispecific antibodies based on CD47 antibody therapies.

Non-human origin antibodies are often immunogenic, which can limit their efficacy and, in some cases, raise concerns about inducing adverse allergic reactions. The immune response to these foreign antibodies promotes their elimination from the body and suppresses their ability to bind to targeted antigens, thereby substantially reducing the effectiveness of the antibodies. To overcome these issues, it is possible to humanize non-human antibodies to decrease immunogenicity in humans while retaining the specificity and affinity of the parental non-human antibody.

In this regard, the present inventors have developed a humanized antibody targeting the immune checkpoint proteins PD-L1 and CD47 as an immune checkpoint inhibitor, leading to the present application.

### [Prior Art Documents]

### [Patent Documents]

US Patent Publication No. 2020-0181259
PCT Patent Publication No. 2011-143624
US Registered Patent No. 8,008,449

### [Non-Patent literature]

Pol Specenier (2016): Ipilimumab in melanoma, Expert Review of Anticancer Therapy.

DB Johnson, C Peng et al. Nivolumab in melanoma: latest evidence and clinical potential. Ther Adv Med Oncol 2015, Vol. 7(2) 97-106

Liu J, Wang L, Zhao F, Tseng S, Narayanan C, Shura L, et al. (2015) Pre-Clinical Development of a Humanized Anti-CD47 Antibody with Anti-Cancer Therapeutic Potential. PLoS ONE 10(9): e0137345.

C Vincke et al. General Strategy to Humanize a Camelid Single-domain Antibody and Identification of a Universal Humanized Nanobody Scaffold. The Journal of Biological Chemistry Vol. 284, NO. 5, 3273-3284, January 30, 2009.

### [Detailed Description of the Invention]

### [Technical Problem]

The purpose of the present invention is to provide a bispecific humanized single domain antibody targeting immune checkpoint proteins PD-L1 and CD47, and uses thereof.

### [Solution to Problem]

To achieve the purpose of the present invention, the present invention provides a bispecific antibody that binds bispecifically to PD-L1 and CD47, comprising: a first humanized single domain antibody (first hsdAb) or an antigen-binding fragment thereof, which specifically binds to PD-L1; and a second humanized single domain antibody (second hsdAb) or an antigen-binding fragment thereof, which specifically binds to CD47.

In one aspect of the present invention, the first hsdAb or an antigen-binding fragment thereof may comprise a CDR1 consisting of an amino acid sequence represented by SEQ ID NO: 2; a CDR2 consisting of an amino acid sequence represented by SEQ ID NO: 3; and a CDR3 consisting of an amino acid sequence represented by SEQ ID NO: 4.

Additionally, the second hsdAb or an antigen-binding fragment thereof may comprise a CDR1 consisting of an amino acid sequence represented by SEQ ID NO: 9, a CDR2 consisting of an amino acid sequence represented by SEQ ID NO: 10, and a CDR3 consisting of an amino acid sequence represented by SEQ ID NO: 11.

Additionally, the first hsdAb or the second hsdAb may comprise a heavy chain variable domain framework sequence having at least 95% sequence identity to the sequence of SEQ ID NO: 16, and may comprise a heavy chain variable domain framework sequence having at least 85% sequence identity to the sequence of SEQ ID NO: 17.

Additionally, the first hsdAb or the second hsdAb may comprise a heavy chain variable domain framework sequence having 100% sequence identity to the sequence of SEQ ID NO: 16 or SEQ ID NO: 17. Specifically, the first hsdAb or an antigen-binding fragment thereof and/or the second hsdAb or an antigen-binding fragment thereof may comprise a heavy chain variable domain framework sequence comprising: (1) a FR1 consisting of an amino acid sequence represented by either SEQ ID NO: 19 or SEQ ID NO: 23; (2) a FR2 consisting of an amino acid sequence represented by either SEQ ID NO: 20 or SEQ ID NO: 24; (3) a FR3 consisting of an amino acid sequence represented by either SEQ ID NO: 21 or SEQ ID NO: 25; and (4) a FR4 consisting of an amino acid sequence represented by either SEQ ID NO: 22 or SEQ ID NO: 26. More specifically, the first hsdAb or an antigen-binding fragment thereof may comprise a heavy chain variable domain framework sequence comprising a FR1 consisting of an amino acid sequence represented by SEQ ID NO: 19, a FR2 consisting of an amino acid sequence represented by SEQ ID NO: 20, a FR3 consisting of an amino acid sequence represented by SEQ ID NO: 21, and a FR4 consisting of an amino acid sequence represented by SEQ ID NO: 22, while the second hsdAb or an antigen-binding fragment thereof may comprise a heavy chain variable domain framework sequence comprising a FR1 consisting of an amino acid sequence represented by SEQ ID NO: 23, a FR2 consisting of an amino acid sequence represented by SEQ ID NO: 24, a FR3 consisting of an amino acid sequence represented by SEQ ID NO: 25, and a FR4 consisting of an amino acid sequence represented by SEQ ID NO: 26. In certain embodiments, the first hsdAb comprises an amino acid sequence represented by SEQ ID NO: 16, and the second hsdAb comprises an amino acid sequence represented by SEQ ID NO: 17.

Additionally, the first hsdAb or an antigen-binding fragment thereof and/or the second hsdAb or an antigen-binding fragment thereof may be monovalent, divalent, trivalent, tetravalent, or of higher valency. Furthermore, the first hsdAb or an antigen-binding fragment thereof may be fused to each other through a peptide linker. Additionally, the second hsdAb or an antigen-binding fragment thereof may be fused to each other through a peptide linker.

In one aspect of the present invention, the first hsdAb or an antigen-binding fragment thereof and the second hsdAb or an antigen-binding fragment thereof may be fused to each other through a peptide linker, and in certain embodiments, they comprise an amino acid sequence represented by SEQ ID NO: 15.

In one aspect of the present invention, a heavy chain-only antibody (HCAb) is provided, wherein an Fc fragment is fused to the first hsdAb or an antigen-binding fragment thereof, or to the second hsdAb or an antigen-binding fragment thereof.

In one aspect of the present invention, a bispecific and multivalent heavy chain-only antibody (HCAb) is provided, comprising two or more copies of the first hsdAb or an antigen-binding fragment thereof and/or the second hsdAb or an antigen-binding fragment thereof, wherein the HCAb may be bivalent, trivalent, tetravalent, or of higher valency, and wherein an Fc fragment is fused to the HCAb. In certain embodiments, the HCAb may consist of an amino acid sequence represented by SEQ ID NO: 18.

In one aspect of the present invention, the sdAb may be fused to the Fc fragment through a peptide linker, and the Fc fragment may be human IgG1, IgG2, IgG3, or IgG4.

In one aspect of the present invention, the HCAb may comprise at least one amino acid substitution, wherein the at least one amino acid substitution may be a conservative substitution and may be a substitution with a non-genetic coding amino acid or a synthetic amino acid.

In one aspect of the present invention, an immunomodulator, cytokine, cytotoxic agent, chemotherapeutic agent, diagnostic agent, antiviral agent, antimicrobial agent, or drug may be conjugated. Accordingly, the present invention provides an antibody conjugate comprising the bispecific antibody that binds bispecifically to PD-L1 and CD47, conjugated to an immunomodulator, cytokine, cytotoxic agent, chemotherapeutic agent, diagnostic agent, antiviral agent, antimicrobial agent, or drug.

Additionally, the present invention provides a nucleic acid molecule encoding the bispecific antibody that binds bispecifically to PD-L1 and CD47.

Additionally, the present invention provides an expression vector comprising the nucleic acid molecule.

Additionally, the present invention provides a host cell transformed with the expression vector.

Additionally, the present invention provides a method for producing a bispecific antibody that binds bispecifically to PD-L1 and CD47, comprising the steps of:
(a) culturing the host cell under conditions that allow for the expression of the bispecific antibody; and
(b) recovering the expressed bispecific antibody.

Additionally, the present invention provides a pharmaceutical composition for the prevention or treatment of cancer, comprising as an active ingredient the above bispecific antibody that binds bispecifically to PD-L1 and CD47 or the above antibody conjugate; a method for the prevention or treatment of cancer, comprising the step of administering the bispecific antibody that binds bispecifically to PD-L1 and CD47, or the antibody conjugate to an individual; the use of the bispecific antibody that binds bispecifically to PD-L1 and CD47, or the antibody conjugate for the prevention or treatment of cancer; and the use of the bispecific antibody that binds bispecifically to PD-L1 and CD47, or the antibody conjugate for the manufacture of a composition for the prevention or treatment of cancer.

In one aspect of the present invention, the cancer may be selected from the group consisting of melanoma, lung cancer, liver cancer, glioblastoma, ovarian cancer, colorectal cancer, head and neck cancer, bladder cancer, renal cell carcinoma, gastric cancer, breast cancer, metastatic cancer, prostate cancer, pancreatic cancer, non-Hodgkin lymphoma, Hodgkin lymphoma, multiple myeloma, leukemia, lymphoma, myelodysplastic syndrome, acute lymphoblastic leukemia, acute myeloid leukemia, chronic lymphocytic leukemia, chronic myeloid leukemia, solitary plasmacytoma, and aplastic anemia.

In one aspect of the present invention, the pharmaceutical composition may further comprise a pharmaceutically acceptable carrier.

### [Advantageous Effects of Invention]

In the present invention, a humanized single-domain antibody that binds bispecifically to the immune checkpoint proteins PD-L1 and CD47 has been developed, and its *in vitro* and *in vivo* efficacy has been confirmed. Therefore, the bispecific humanized single-domain antibody can be advantageously utilized as an immune checkpoint inhibitor in immuno-oncology therapies.

### [Brief Description of Drawings]

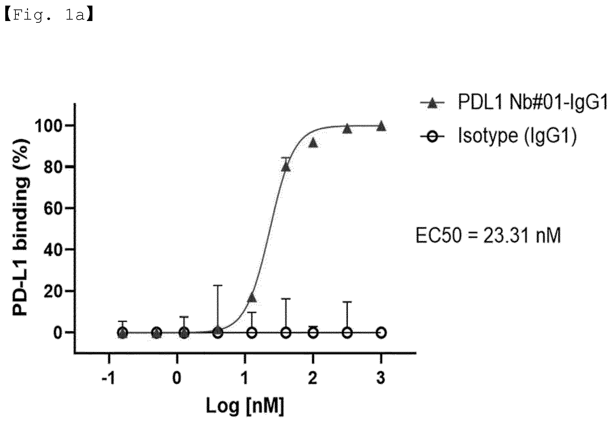

Fig. 1a illustrates the binding affinity (EC50) of the anti-PD-L1 HCAb (PDL1 Nb#01-IgG1) produced according to an embodiment of the present invention to the PD-L1 antigen expressed on the cell surface. This binding affinity was confirmed by reacting the anti-PD-L1 HCAb with a CHO-K1_PD-L1 cell line (CHO-K1 cells induced to express the PD-L1 antigen) at varying concentrations.

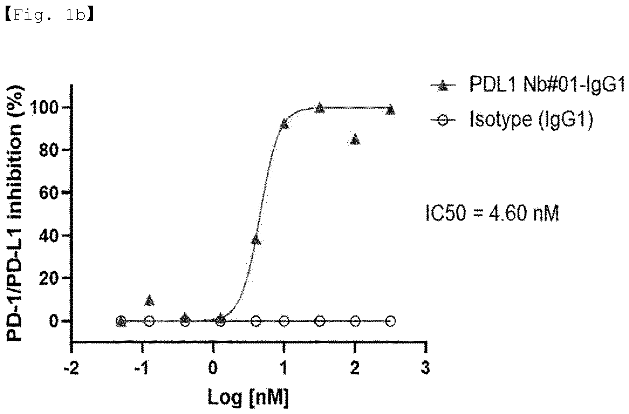

Fig. 1b illustrates the inhibitory activity (IC50) of the anti-PD-L1 HCAb (PDL1 Nb#01-IgG1), produced according to an embodiment of the present invention, on the PD-L1/PD-1 interaction. This inhibitory activity was confirmed by evaluating the binding of the anti-PD-L1 HCAb to a CHO-K1_PD-L1 cell line (CHO-K1 cells induced to express the PD-L1 antigen) following the binding of the PD-1 protein.

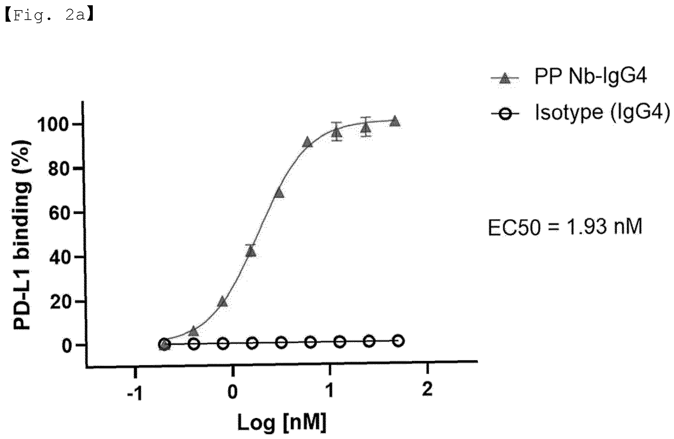

Fig. 2a illustrates the binding affinity (EC50) of the bivalent anti-PD-L1 HCAb (PP Nb-IgG4), produced according to an embodiment of the present invention, to the PD-L1 antigen expressed on the cell surface. This binding affinity was confirmed by reacting the bivalent anti-PD-L1 HCAb with a CHO-K1_PD-L1 cell line (CHO-K1 cells induced to express the PD-L1 antigen) at varying concentrations.

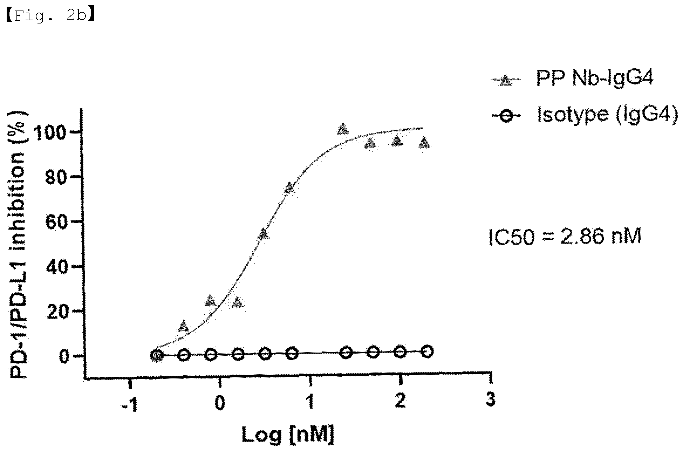

Fig. 2b illustrates the inhibitory activity (IC50) of the bivalent anti-PD-L1 HCAb (PP Nb-IgG4), produced according to an embodiment of the present invention, on the PD-L1/PD-1 interaction. This inhibitory activity was confirmed by evaluating the binding of the bivalent anti-PD-L1 HCAb to a CHO-K1_PD-L1 cell line (CHO-K1 cells induced to express the PD-L1 antigen) following the binding of the PD-1 protein.

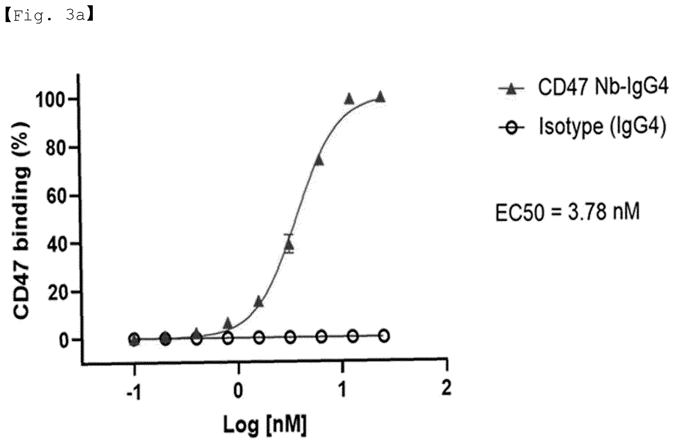

Fig. 3a illustrates the binding affinity (EC50) of the anti-CD47 HCAb (CD47 Nb-IgG4), produced according to an embodiment of the present invention, to the CD47 antigen expressed on the cell surface. This binding affinity was confirmed by reacting the anti-CD47 HCAb with an Expi-CHO_CD47 cell line (Expi-CHO cells induced to express the CD47 antigen) at varying concentrations.

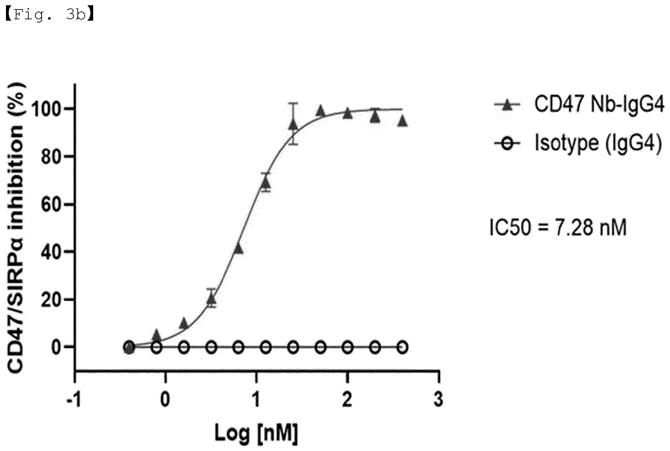

Fig. 3b illustrates the inhibitory activity (IC50) of the anti-CD47 HCAb (CD47 Nb-IgG4), produced according to an embodiment of the present invention, on the CD47/SIRPalpha interaction. This inhibitory activity was confirmed by evaluating the binding of the anti-CD47 HCAb to an Expi-CHO_CD47 cell line (Expi-CHO cells induced to express the CD47 antigen) following the binding of the SIRPalpha protein.

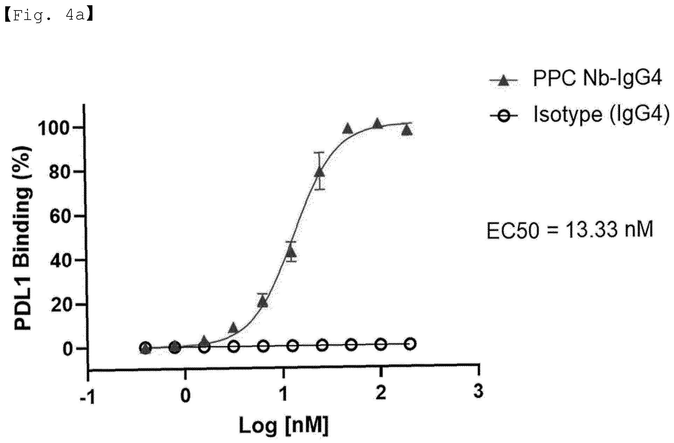

Fig. 4a illustrates the binding affinity (EC50) of the anti-PD-L1×CD47 HCAb (PPC Nb-IgG4), prepared according to an embodiment of the present invention, to the PD-L1 antigen expressed on the cell surface. This binding affinity was confirmed by reacting the anti-PD-L1×CD47 HCAb with a CHO-K1_PD-L1 cell line (CHO-K1 cells induced to express the PD-L1 antigen) at varying concentrations.

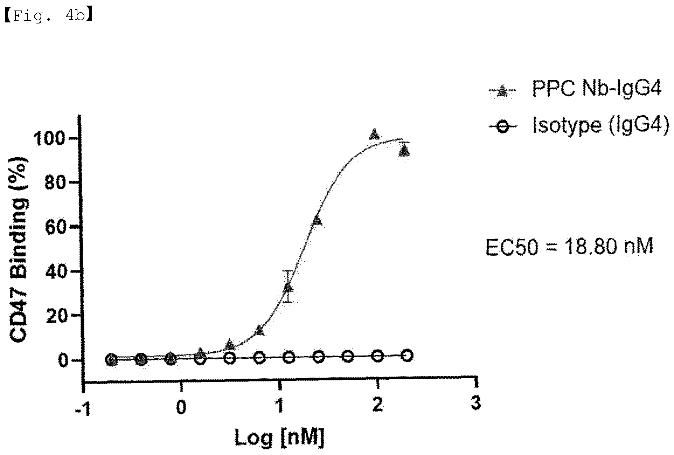

Fig. 4b illustrates the binding affinity (EC50) of the anti-PD-L1×CD47 HCAb (PPC Nb-IgG4), produced according to an embodiment of the present invention, to the CD47 antigen expressed on the cell surface. This binding affinity was confirmed by reacting the anti-PD-L1×CD47 HCAb with an Expi-CHO_CD47 cell line (Expi-CHO cells induced to express the CD47 antigen) at varying concentrations.

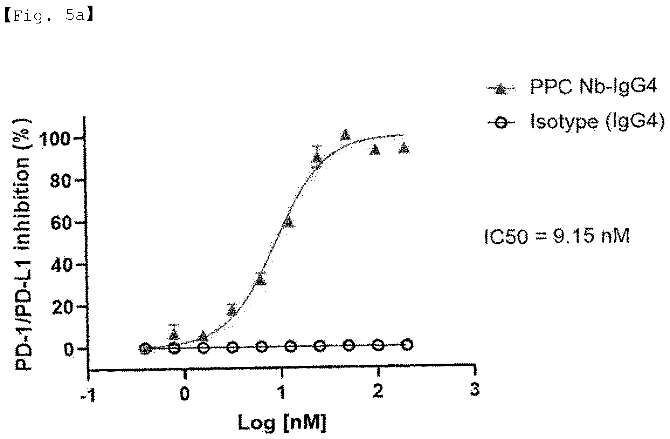

Fig. 5a illustrates the inhibitory activity (IC50) of the anti-PD-L1×CD47 HCAb (PPC Nb-IgG4), produced according to an embodiment of the present invention, on the PD-L1/PD-1 interaction. This inhibitory activity was confirmed by evaluating the binding of the anti-PD-L1×CD47 HCAb to a CHO-K1_PD-L1 cell line (CHO-K1 cells induced to express the PD-L1 antigen) following the binding of the PD-1 protein.

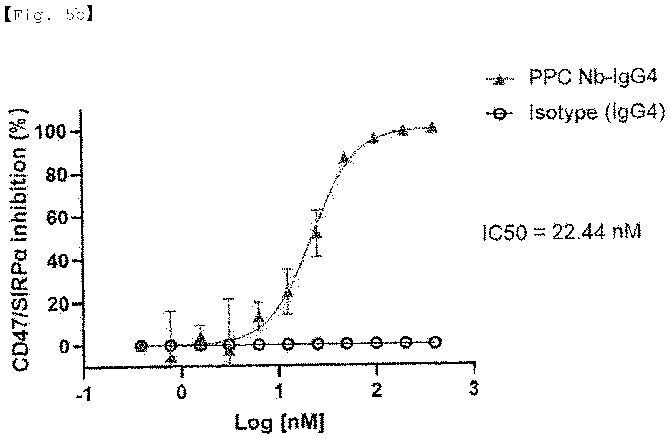

Fig. 5b illustrates the inhibitory activity (IC50) of the anti-PD-L1×CD47 HCAb (PPC Nb-IgG4), produced according to an embodiment of the present invention, on the CD47/SIRPalpha interaction. This inhibitory activity was confirmed by evaluating the binding of the anti-PD-L1×CD47 HCAb to an Expi-CHO_CD47 cell line (Expi-CHO cells induced to express the CD47 antigen) following the binding of the SIRPalpha protein.

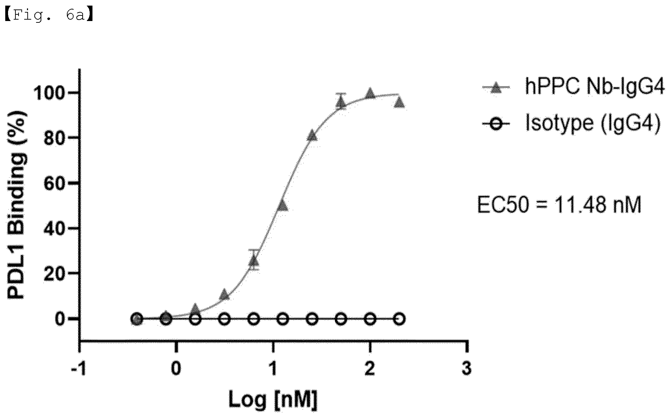

Fig. 6a illustrates the binding affinity (EC50) of the humanized anti-PD-L1×CD47 HCAb (hPPC Nb-IgG4), produced according to an embodiment of the present invention, to the PD-L1 antigen expressed on the cell surface. This binding affinity was confirmed by reacting the humanized anti-PD-L1×CD47 HCAb with a CHO-K1_PD-L1 cell line (CHO-K1 cells induced to express the PD-L1 antigen) at varying concentrations.

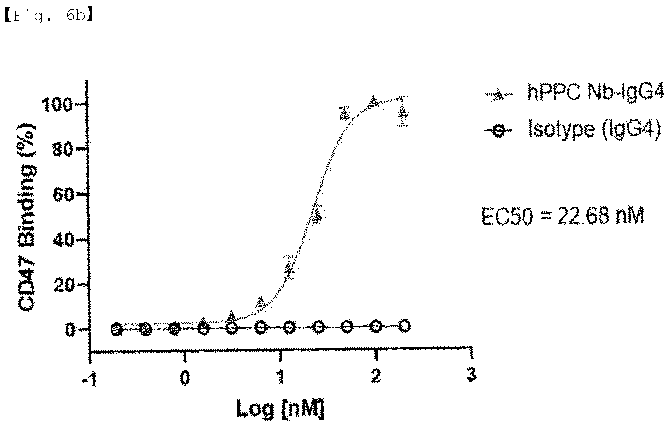

Fig. 6b illustrates the binding affinity (EC50) of the humanized anti-PD-L1×CD47 HCAb (hPPC Nb-IgG4), produced according to an embodiment of the present invention, to the CD47 antigen expressed on the cell surface. This binding affinity was confirmed by reacting the humanized anti-PD-L1×CD47 HCAb with an Expi-CHO_CD47 cell line (Expi-CHO cells induced to express the CD47 antigen) at varying concentrations.

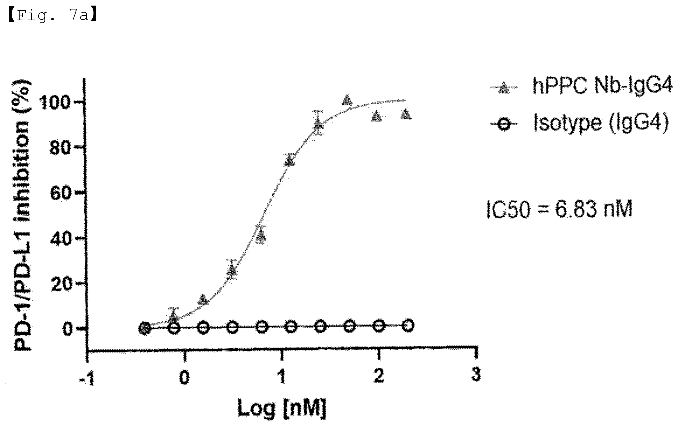

Fig. 7a illustrates the inhibitory activity (IC50) of the humanized anti-PD-L1×CD47 HCAb (hPPC Nb-IgG4), produced according to an embodiment of the present invention, on the PD-L1/PD-1 interaction. This inhibitory activity was confirmed by evaluating the binding of the humanized anti-PD-L1×CD47 HCAb to a CHO-K1_PD-L1 cell line (CHO-K1 cells induced to express the PD-L1 antigen) following the binding of the PD-1 protein.

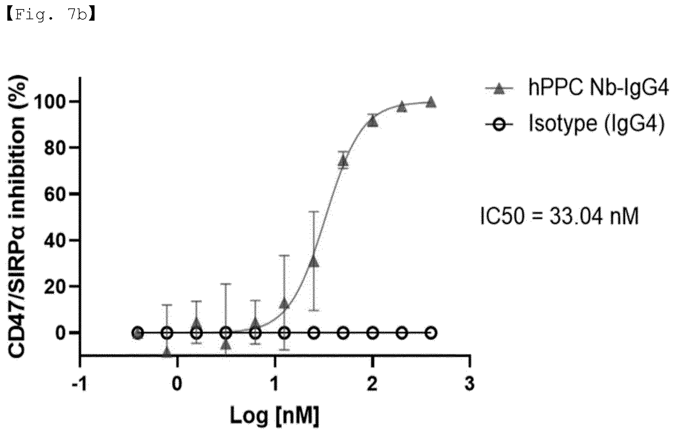

Fig. 7b illustrates the inhibitory activity (IC50) of the humanized anti-PD-L1×CD47 HCAb (hPPC Nb-IgG4), produced according to an embodiment of the present invention, on the CD47/SIRPalpha interaction. This inhibitory activity was confirmed by evaluating the binding of the humanized anti-PD-L1×CD47 HCAb to an Expi-CHO_CD47 cell line (Expi-CHO cells induced to express the CD47 antigen) following the binding of the SIRPalpha protein.

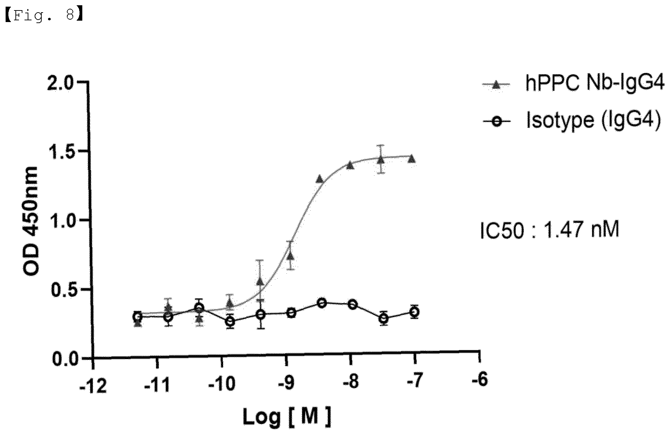

Fig. 8 illustrates the T cell activation induced by the interference of cell-to-cell interaction through the humanized anti-PD-L1×CD47 HCAb (hPPC Nb-IgG4), produced according to an embodiment of the present invention. This was confirmed using CHO-K1 cells, which induce the overexpression of PD-L1 protein, and Jurkat cells, which express PD-1.

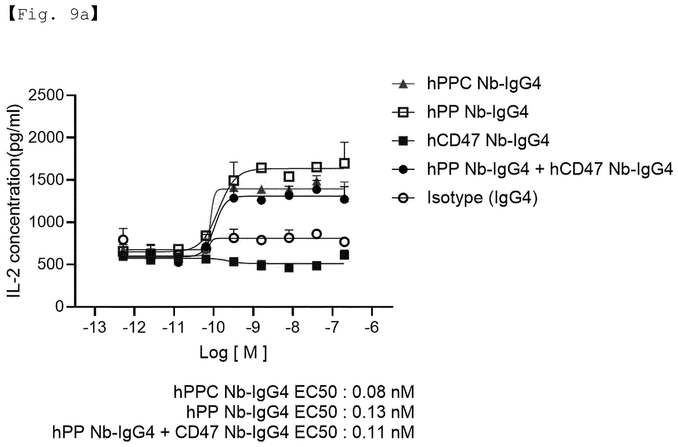

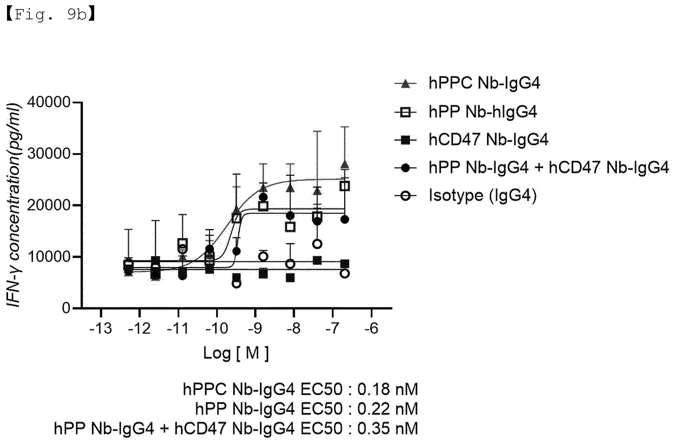

Figs. 9a and 9b illustrate the activation of CD4⁺ T cells in a mixed lymphocyte reaction, confirming the degree of activation induced by the humanized anti-PD-L1×CD47 HCAb (hPPC Nb-IgG4), produced according to an embodiment of the present invention.

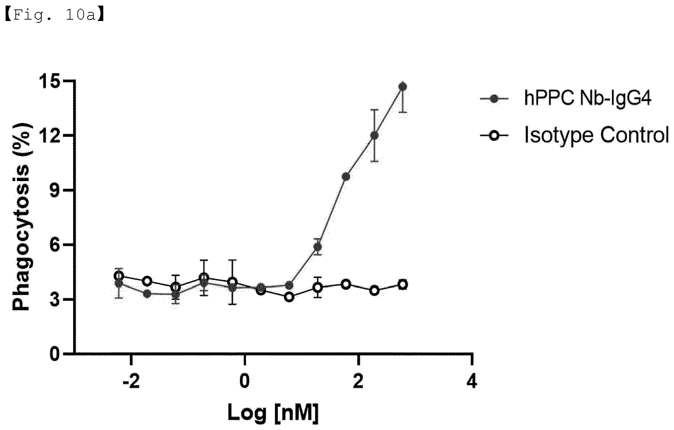

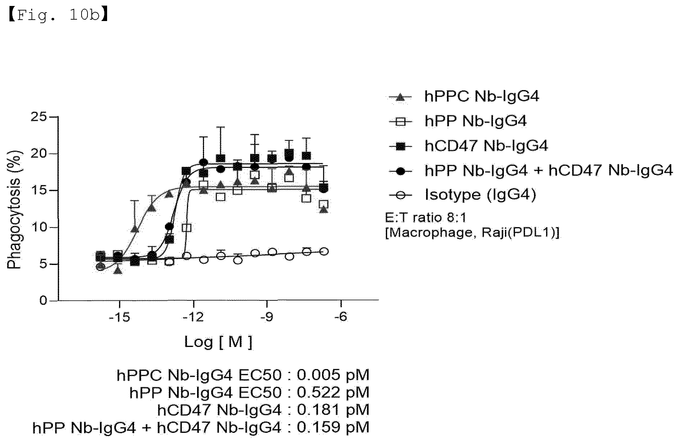

Figs. 10a and 10b illustrate the degree of phagocytosis by macrophages, confirming the phagocytic activity induced by the humanized anti-PD-L1×CD47 HCAb (hPPC Nb-IgG4), produced according to an embodiment of the present invention.

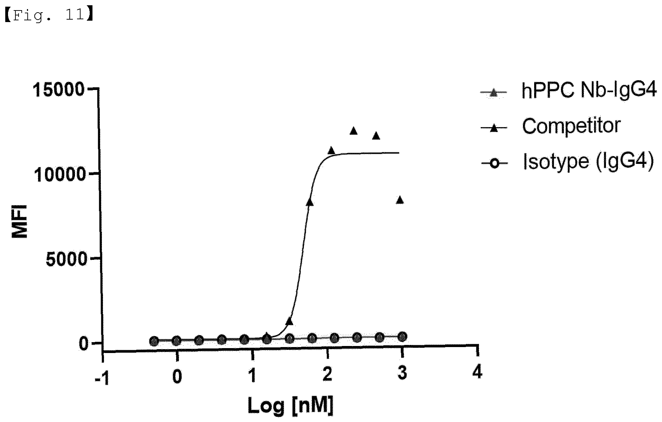

Fig. 11 illustrates the binding ability of the humanized anti-PD-L1×CD47 HCAb (hPPC Nb-IgG4) to human RBCs (red blood cells), produced according to an embodiment of the present invention.

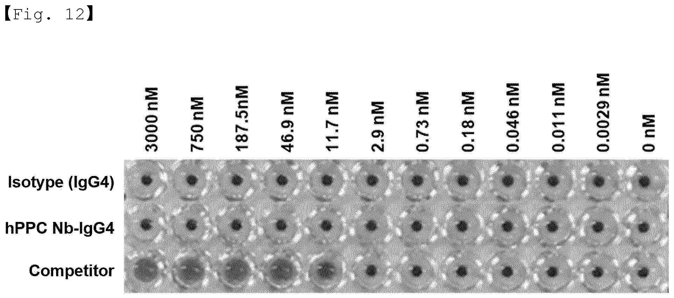

Fig. 12 illustrates the hemagglutination response of the humanized anti-PD-L1×CD47 HCAb (hPPC Nb-IgG4), produced according to an embodiment of the present invention.

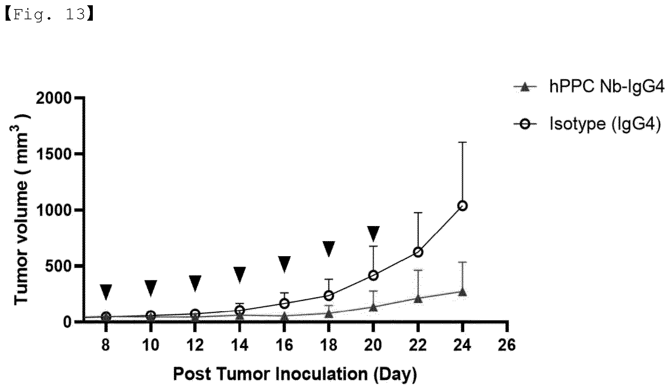

Fig. 13 illustrates the antitumor effect of the humanized anti-PD-L1×CD47 HCAb (hPPC Nb-IgG4) in a PD-L1 and CD47 antigen-expressing tumor-bearing C57BL/6 mouse model.

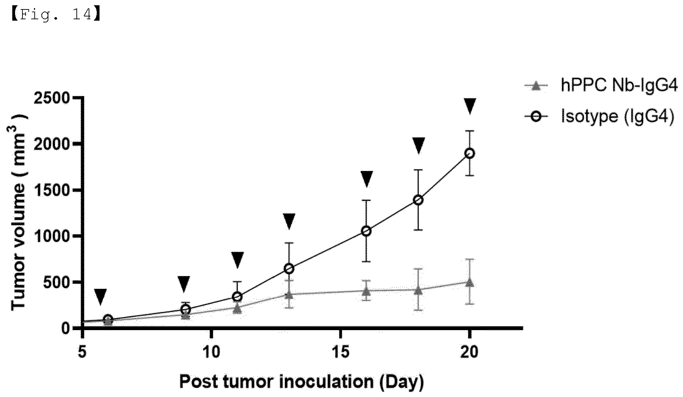

Fig. 14 illustrates the antitumor effect of the humanized anti-PD-L1×CD47 HCAb (hPPC Nb-IgG4) in a PD-L1 and CD47 antigen-expressing tumor-bearing humanized NSG mouse model.

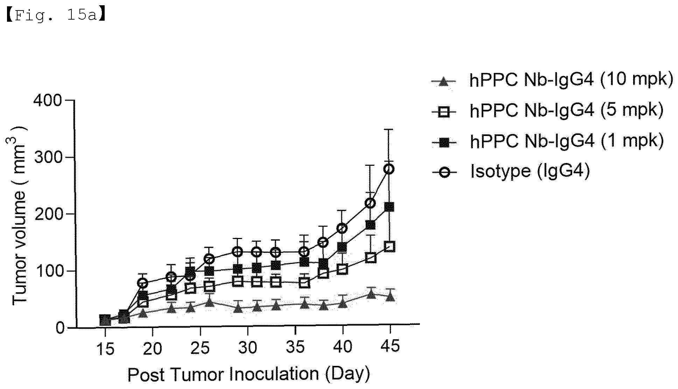

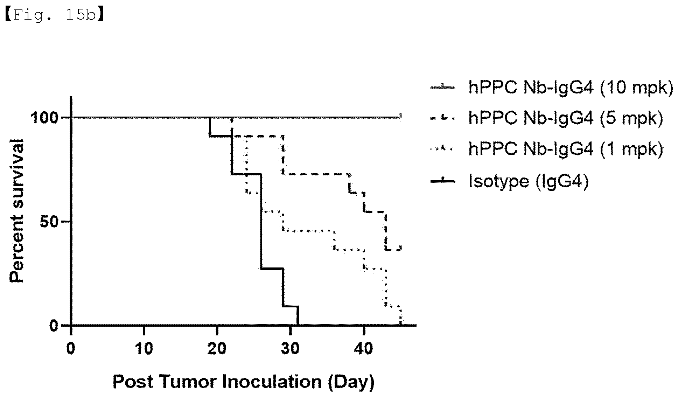

Figs. 15a and 15b illustrate the antitumor effect and survival rate of the humanized anti-PD-L1×CD47 HCAb (hPPC Nb-IgG4) in a humanized NSG mouse model with tumor formation induced by the human breast cancer cell line MDA-MB-231.

### [Best Mode for Carrying Out the Invention]

The embodiments of the present invention are described in detail below to enable those skilled in the art to easily carry out the invention. These embodiments are provided to more fully explain the present invention to a person of ordinary skill in the art. Therefore, the embodiments of the present invention may be modified in various ways, and the scope of the present invention is not limited to the embodiments described below.

In the present invention, the term "epitope" refers to a protein determinant that can specifically bind to an antibody. An epitope typically consists of chemically active surface groupings of molecules, such as amino acids or sugar side chains, and generally has specific three-dimensional structural characteristics, as well as specific charge characteristics.

The term "treatment" refers to any process that alleviates, interrupts, halts, controls, stops, relieves, or improves the symptoms or complications of a disorder or disease disclosed herein, or reverses its progression, but does not necessarily imply the complete elimination of all symptoms of the disease or disorder.

The term "prevention" refers to the prophylactic treatment of a disease or disorder, or the delay of the onset or progression of the disease or disorder.

The term "subject" or "individual" refers, without limitation, to a mammal, including humans, cattle, horses, cats, dogs, rodents, or primates. In certain embodiments, the subject is a human.

The term "antibody" is used in its broadest sense and encompasses various antibody structures, including but not limited to monoclonal antibodies, polyclonal antibodies, multispecific antibodies (e.g., bispecific antibodies), full-length antibodies, and their antigen-binding fragments, as long as they exhibit the desired antigen-binding activity. The term "antibody" also includes conventional four-chain antibodies, single-domain antibodies, and their antigen-binding fragments.

The basic four-chain antibody unit is a heterotetrameric glycoprotein consisting of two identical light (L) chains and two identical heavy (H) chains. IgM antibodies consist of five of these basic heterotetrameric units, together with an additional polypeptide known as the J chain, and contain ten antigen-binding sites. In contrast, IgA antibodies include two to five basic four-chain units that can polymerize to form multimeric assemblies in combination with the J chain. In the case of IgG, the four-chain unit typically has a molecular weight of approximately 150,000 Daltons. Each L chain is linked to the H chain by a single covalent disulfide bond, while the two H chains are connected to each other by one or more disulfide bonds, depending on the H chain isotype. Each H and L chain also has inter-chain disulfide bridges regularly spaced. Each H chain contains, at the N-terminus, a variable domain (VH) for each of the α and γ chains, followed by three constant domains (CH), and four CH domains for the µ and ε isotypes. Each L chain contains, at the N-terminus, a variable domain (VL) at its other terminus, followed by a constant domain. The VL aligns with the VH, and the CL aligns with the first constant domain of the heavy chain (CH1). The pairing of VH and VL forms a single antigen-binding site. The L chain from any vertebrate species can be assigned to one of two distinctly defined types, called kappa and lambda, based on the amino acid sequence of their constant domains. Immunoglobulins can be assigned to different classes or isotypes based on the amino acid sequence of the constant domain (CH) of their heavy chains. There are five classes of immunoglobulins: IgA, IgD, IgE, IgG, and IgM, with heavy chains designated α, δ, ε, γ, and µ, respectively. The γ and α classes are further divided into subclasses based on relatively minor differences in CH sequence and function, for example, humans express the following subclasses: IgG1, IgG2A, IgG2B, IgG3, IgG4, IgA1 and IgA2.

The term "heavy chain-only antibody" or "HCAb" refers to a functional antibody that contains a heavy chain but lacks the light chain typically found in a conventional four-chain antibody.

The term "single-domain antibody," "nanobody," or "sdAb" refers to a single antigen-binding polypeptide that contains three complementarity-determining regions (CDRs). An sdAb alone can bind to an antigen without pairing with the corresponding CDR-containing polypeptide. In some cases, the single-domain antibody is engineered from the camelid HCAb, where their heavy chain variable domain is referred to as "VHH" (variable domain of the heavy chain of the heavy chain antibody) in the present application. The basic VHH has the following structure from the N-terminus to the C-terminus: FR1-CDR1-FR2-CDR2-FR3-CDR3-FR4, where FR1 to FR4 refer to framework regions 1 to 4, respectively, and CDR1 to CDR3 refer to complementarity-determining regions 1 to 3.

The "variable region" or "variable domain" of an antibody refers to the amino-terminal domain of the antibody's heavy chain or light chain. The variable domains of the heavy chain and light chain are commonly referred to as "VH" and "VL," respectively. These domains are generally the most variable parts of the antibody (compared to other antibodies of the same class) and contain the antigen-binding site. Heavy chain-only antibodies from camelid species have a single heavy-chain variable domain, referred to as "VHH."

The term "variable" refers to the fact that specific segments of the variable domains exhibit significant sequence diversity among antibodies. The V domains mediate antigen binding and define the specificity of a particular antibody toward its specific antigen. However, variability is not evenly distributed across the entire range of the variable domain. Instead, it is concentrated in three segments known as the complementary determining regions (CDR) or hypervariable regions (HVR) in both the heavy and light chain variable domains. The more highly conserved regions of the variable domain are referred to as the framework regions (FR). The variable domains of the native heavy and light chains each include four framework (FR) regions, which primarily adopt a beta-sheet structure and, in some cases, form part of the beta-sheet structure, with three CDRs connected by loop linkages. The CDRs in each chain are closely maintained together by the FR regions, and the CDRs from the different chains contribute to the formation of the antibody's antigen-binding site (see Kabat, Elvin A., Sequence of Immunological Interest, Fifth Edition, National Institute of Health, Bethesda, Md. (1991)). The constant domains do not directly participate in the binding of the antibody to the antigen, but they are involved in various effector functions, such as mediating antibody-dependent cellular cytotoxicity.

The term "constant domain" refers to the portion of an immunoglobulin molecule that has a more conserved amino acid sequence than the variable domain, the other portion of the immunoglobulin that contains the antigen-binding site. The constant domains contain the CH1, CH2, and CH3 domains of the heavy chain (collectively, referred to as CH) and the CHL (or CL) domain of the light chain.

The term "full-length antibody," "intact antibody," or "whole antibody" is used interchangeably to refer to an antibody in its substantially complete form, as opposed to an antibody fragment. Specifically, a full-length four-chain antibody includes a heavy and light chain, both of which contain an Fc region. A full-length heavy chain-only antibody includes a heavy chain variable domain (e.g., VHH) and an Fc region. The constant domains may be either native sequence constant domains (e.g., human native sequence constant domains) or variants of their amino acid sequences. In some cases, the intact antibody may possess one or more effector functions.

The term "antibody fragment" or "antigen-binding fragment" refers to a portion of an intact antibody, preferably including the antigen-binding and/or variable regions of the intact antibody. Examples of antibody fragments include, but are not limited to, Fab, Fab', F(ab')2, and Fv fragments; diabodies; linear antibodies; single-chain antibodies (scFv) molecules; single-domain antibodies (e.g., VHH); and multispecific antibodies formed from antibody fragments. "Fv" refers to the minimal antibody fragment that contains the complete antigen-recognition and -binding sites. This fragment consists of a dimer of one heavy chain and one light chain variable domain, tightly associated in a non-covalent manner. The term "single-chain Fv," also abbreviated as "sFv" or "scFv," refers to an antibody fragment that includes the VH and VL antibody domains connected in a single polypeptide chain. Preferably, the scFv polypeptide further includes a polypeptide linker between the VH and VL domains, allowing the scFv to adopt the desired structure for antigen binding. The term "diabody" refers to a small antibody fragment produced by constructing an sFv fragment with a short linker (about 5-10 amino acid residues) between the VH and VL domains, which facilitates inter-chain, rather than intra-chain, pairing of the V domains, thereby resulting in a bivalent fragment containing two antigen-binding sites. A bispecific diabody is a heterodimer of two "cross" sFv fragments, where the VH and VL domains of two different antibodies are present on distinct polypeptide chains.

The term "humanized antibody" is used as a subset of "chimeric antibody."

The "humanized" form of a non-human (e.g., llama or camelid) antibody is a chimeric antibody that contains the minimal sequence derived from a non-human immunoglobulin. In some embodiments, the humanized antibody is an immunoglobulin in which residues from the CDR of the donor species (e.g., mouse, rat, rabbit, camel, llama, alpaca, or non-human primate CDRs) are replaced with residues from the CDR of a human immunoglobulin (recipient antibody) that has the desired specificity, affinity, and/or functionality.

In some instances, the framework ("FR") residues of the human immunoglobulin are replaced with corresponding non-human residues. Additionally, the humanized antibody may include residues not found in either the recipient antibody or the donor antibody. These modifications may be implemented to further enhance the antibody's performance, such as improving binding affinity.

The term "hypervariable region," "HVR," or "HV" refers to the region of the antibody variable domain that exhibits hypervariability and/or forms a structurally defined loop, as used herein. Generally, a single-domain antibody includes three HVRs (or CDRs): HVR1 (or CDR1), HVR2 (or CDR2), and HVR3 (or CDR3). HVR3 (or CDR3) is known to exhibit the highest diversity among the three HVRs and plays a unique role in conferring fine specificity to the antibody. For example, see Hamers-Casterman et al., Nature 363:446-448 (1993); Sheriff et al., Nature Struct. Biol. 3:733-736 (1996).

The term "complementarity-determining region" or "CDR" is used to refer to the hypervariable region as defined by the Kabat system. See Kabat, Elvin A., Sequences of Proteins of Immunological Interest, 5th Ed., Public Health Service, National Institutes of Health, Bethesda, Md. (1991). Kabat complementarity-determining region (CDR) is most commonly used based on sequence variability.

The term "framework" or "FR" residues refer to the variable-domain residues other than the HVR residues, as defined herein.

The term "specific" refers to the selective recognition of a particular epitope of an antigen by an antigen-binding protein (e.g., sdAb).

A native antibody is, for example, monospecific. As used herein, the term "multispecific" refers to an antigen-binding protein having polyepitope specificity (i.e., the ability to specifically bind to two, three, or more distinct epitopes on a single biological molecule or the ability to specifically bind to epitopes on two, three, or more distinct biological molecules). As used herein, "bispecific" refers to an antigen-binding protein having two distinct antigen-binding specificities.

As used herein, the term "monospecific" refers to an antigen-binding protein having one or more binding sites, each of which specifically binds to the same epitope of the same antigen.

The term "valent" refers to the specified number of binding sites present in an antigen-binding protein. For example, the terms "divalent," "trivalent," "tetravalent," "pentavalent," and "hexavalent" refer to the presence of two, three, four, five, and six binding sites, respectively, in the antigen-binding protein.

The term "antibody effector function" refers to the biological activities of an antibody attributed to its Fc region (whether from a native sequence Fc region or an amino acid sequence variant of the Fc region), and varies according to the antibody isotype. Examples of antibody effector functions include: C1q binding and complement-dependent cytotoxicity; Fc receptor binding; antibody-dependent cell-mediated cytotoxicity (ADCC); phagocytosis; downregulation of cell surface receptors (e.g., B cell receptors); and B cell activation. 'Complement-dependent cytotoxicity' or 'CDC' refers to the lysis of target cells in the presence of complement. Activation of the classical complement pathway is initiated by the binding of the first component of the complement system (C1q) to antibodies of the appropriate subclass, which bind to their homologous antigens. "Antibody-Dependent Cell-mediated Cytotoxicity" or ADCC refers to a form of cytotoxicity in which secreted Ig bound to Fc receptors (FcR) present in specific cytotoxic cells (e.g., natural killer (NK) cells, neutrophils and macrophages) specifically binds these cytotoxic effector cells to antigen-bearing target cells and subsequently kills the target cell with cytotoxin.

In the present application, the term "Fc region" or "fragment crystallizable region" is used to define the C-terminal region of the immunoglobulin heavy chain, including both the native-sequence Fc region and variants thereof. Suitable native-sequence Fc regions for use in the antibodies described herein include human IgG1, IgG2 (IgG2A, IgG2B), IgG3, and IgG4.

The term "binding affinity" generally refers to the strength of the total non-covalent interactions between a single binding site of a molecule (e.g., an antibody) and its binding partner (e.g., an antigen). Unless otherwise specified, as used herein, "binding affinity" refers to the intrinsic binding affinity that reflects the 1:1 interaction between the members of the binding pair. Binding affinity may be represented by Kd, Koff, Kon, or Ka. As used herein, the term equilibrium dissociation constant "KD" or "Kd" refers to the dissociation constant for a specific antibody-antigen interaction and describes the concentration of antigen required to occupy half of all antibody-binding domains present in solution at equilibrium, expressed in units of M. Measurement of KD assumes that all binding reagents are in solution. The dissociation constant (KD or Kd) serves as an indicator of the antibody's affinity for the antigen. For example, straightforward analysis may be achieved using the Scatchard method with antibodies marked with various marker agents, as well as through the use of over-the-counter measurement kits, according to the user manual and experimental operating procedures attached to the kits. The KD values derived by these methods are expressed in units of M (Molar).

The terms "percent (%) amino acid sequence identity" and "homology" with respect to peptide, polypeptide, or antibody sequences are defined as the percentage of amino acid residues in a candidate sequence that are identical to the amino acid residues in a specified peptide or polypeptide sequence, without taking into account any conservative substitutions as part of the sequence identity, following sequence alignment and gap introduction if necessary to achieve maximum percent sequence identity. For purposes of determining percent amino acid sequence identity, alignment can be achieved using various methods known in the art, for example, by employing publicly available computer software such as BLAST, BLAST-2, ALIGN, or MEGALIGN^{™} (DNATAR) software. A person skilled in the art can determine appropriate parameters for measuring alignment, including any algorithm necessary to achieve optimal alignment across the full length of the sequences to be compared.

The present invention relates to a bispecific antibody that binds bispecifically to PD-L1 and CD47 (hereinafter referred to as "humanized anti-PD-L1×CD47 bsAb"), comprising a first humanized single-domain antibody (a first hsdAb) or an antigen-binding fragment thereof (anti-PD-L1 hsdAb) which specifically binds to PD-11; and a second humanized single-domain antibody (a second hsdAb) or an antigen-binding fragment thereof (anti-CD47 hsdAb) that specifically binds to CD47, such as a humanized anti-PD-L1×CD47 bsAb, specifically a humanized bispecific single-domain antibody wherein the anti-PD-L1 hsdAb and the anti-CD47 hsdAb are fused and a humanized anti-PD-L1×CD47 heavy chain-only antibody (HCAb) (for example, a humanized anti-PD-L1×CD47 bsAb-Fc fusion protein, wherein crystallizable Fc fragment of human immunoglobulin G (IgG) is fused to the anti-PD-L1 hsdAb and/or the anti-CD47 hsdAb), and relates to the preparation and uses thereof.

Accordingly, the present invention provides a bispecific antibody that binds bispecifically to PD-L1 and CD47, comprising a humanized anti-PD-L1×CD47 bsAb.

In the present invention, the bispecific antibody that binds bispecifically to PD-L1 and CD47, comprising the humanized anti-PD-L1×CD47 bsAb, may be a humanized anti-PD-L1×CD47 bsAb in which a first humanized single-domain antibody (first hsdAb) or an antigen-binding fragment thereof (hereinafter referred to as "anti-PD-L1 hsdAb"), which specifically binds to PD-L1 as a first antigen-binding portion, and a second humanized single-domain antibody (second hsdAb) or an antigen-binding fragment thereof (hereinafter referred to as "anti-CD47 hsdAb"), which specifically binds to CD47 as a second antigen-binding portion, are fused.

In the present invention, the anti-PD-L1 hsdAb comprises CDR1 consisting of the amino acid sequence represented by SEQ ID NO: 2; CDR2 consisting of the amino acid sequence represented by SEQ ID NO: 3; and CDR3 consisting of the amino acid sequence represented by SEQ ID NO: 4.

Additionally, the anti-CD47 hsdAb comprises CDR1 consisting of the amino acid sequence represented by SEQ ID NO: 9; CDR2 consisting of the amino acid sequence represented by SEQ ID NO: 10; and CDR3 consisting of the amino acid sequence represented by SEQ ID NO: 11.

The CDR sequences are provided in Table 6 and Table 12.

In the present invention, the humanized anti-PD-L1×CD47 bsAb includes a human or humanized heavy chain domain framework region. The human or humanized heavy chain domain framework is a framework comprising the amino acid sequence of a heavy chain variable domain (VH) framework derived from a human immunoglobulin framework. The heavy chain variable domain (VH) framework derived from a human immunoglobulin framework may include the same amino acid sequence or contain amino acid substitutions. In certain embodiments, the number of amino acid changes is 10 or fewer, 9 or fewer, 8 or fewer, 7 or fewer, 6 or fewer, 5 or fewer, 4 or fewer, 3 or fewer, or 2 or fewer.

Specifically, the heavy chain variable domain framework sequence may include a sequence that has at least 95%, 96%, 97%, 98%, 99%, or 100% sequence identity to SEQ ID NO: 16. Alternatively, it may include a sequence that has at least 85%, 86%, 87%, 88%, 89%, 90%, 91%, 92%, 93%, 94%, 95%, 96%, 97%, 98%, 99%, or 100% sequence identity to SEQ ID NO: 17.

The heavy chain variable domain framework (FR) sequence having 100% sequence identity to SEQ ID NO: 16 or SEQ ID NO: 17 is provided in Tables 1 to 4.

**[Table 1]**

| No | FR1 | Sequence Number |
|---|---|---|
| 1 | EVQLVESGGGLVQPGGSLRLSCAAS | 19 |
| 2 | EVQLVESGGGLVQPGGSLNLSCAVS | 23 |

**[Table 2]**

| No | FR2 | Sequence Number |
|---|---|---|
| 1 | MSWVRQAPGKGLEWVSD | 20 |
| 2 | VGWARQVPGKGLERVSR | 24 |

**[Table 3]**

| No | FR3 | Sequence Number |
|---|---|---|
| 1 | DYADSVKGRFTISRDNAKNTLYLQMNSLKPEDTAVYYC | 21 |
| 2 | EYLDAVKGRFTISRDNAKNTVYLQMNSLKTEDTAVYYC | 25 |

**[Table 4]**

| No | FR4 | Sequence Number |
|---|---|---|
| 1 | RGQGTQVTVSS | 22 |
| 2 | WGQGTQVTVSS | 26 |

Specifically, the anti-PD-L1 hsdAb may comprise the following FR1, FR2, FR3, and FR4:
FR1 consisting of the amino acid sequence represented by either SEQ ID NO: 19 or SEQ ID NO: 23;
FR2 consisting of the amino acid sequence represented by either SEQ ID NO: 20 or SEQ ID NO: 24;
FR3 consisting of the amino acid sequence represented by either SEQ ID NO: 21 or SEQ ID NO: 25; and
FR4 consisting of the amino acid sequence represented by either SEQ ID NO: 22 or SEQ ID NO: 26.

More specifically, the anti-PD-L1 hsdAb may comprise the following FR1, FR2, FR3, and FR4:
(1) FR1 consisting of the amino acid sequence represented by SEQ ID NO: 19;
   FR2 consisting of the amino acid sequence represented by SEQ ID NO: 20;
   FR3 consisting of the amino acid sequence represented by SEQ ID NO: 21; and
   FR4 consisting of the amino acid sequence represented by SEQ ID NO: 22; or
(2) FR1 consisting of the amino acid sequence represented by SEQ ID NO: 23;
   FR2 consisting of the amino acid sequence represented by SEQ ID NO: 24;
   FR3 consisting of the amino acid sequence represented by SEQ ID NO: 25; and
   FR4 consisting of the amino acid sequence represented by SEQ ID NO: 26.

More specifically, the anti-PD-L1 hsdAb may comprise the following FR1, FR2, FR3, and FR4:
FR1 consisting of the amino acid sequence represented by SEQ ID NO: 19;
FR2 consisting of the amino acid sequence represented by SEQ ID NO: 20;
FR3 consisting of the amino acid sequence represented by SEQ ID NO: 21; and
FR4 consisting of the amino acid sequence represented by SEQ ID NO: 22.

Additionally, the anti-CD47 hsdAb may comprise the following FR1, FR2, FR3, and FR4:
FR1 consisting of the amino acid sequence represented by SEQ ID NO: 19 or SEQ ID NO: 23;
FR2 consisting of the amino acid sequence represented by SEQ ID NO: 20 or SEQ ID NO: 24;
FR3 consisting of the amino acid sequence represented by SEQ ID NO: 21 or SEQ ID NO: 25; and
FR4 consisting of the amino acid sequence represented by SEQ ID NO: 22 or SEQ ID NO: 26.

More specifically, the anti-CD47 hsdAb may comprise the following FR1, FR2, FR3, and FR4:
(1) FR1 consisting of the amino acid sequence represented by SEQ ID NO: 19;
   FR2 consisting of the amino acid sequence represented by SEQ ID NO: 20;
   FR3 consisting of the amino acid sequence represented by SEQ ID NO: 21; and
   FR4 consisting of the amino acid sequence represented by SEQ ID NO: 22; or
(2) FR1 consisting of the amino acid sequence represented by SEQ ID NO: 23;
   FR2 consisting of the amino acid sequence represented by SEQ ID NO: 24;
   FR3 consisting of the amino acid sequence represented by SEQ ID NO: 25; and
   FR4 consisting of the amino acid sequence represented by SEQ ID NO: 26.

More specifically, the anti-CD47 hsdAb may comprise the following FR1, FR2, FR3, and FR4:
FR1 consisting of the amino acid sequence represented by SEQ ID NO: 23;
FR2 consisting of the amino acid sequence represented by SEQ ID NO: 24;
FR3 consisting of the amino acid sequence represented by SEQ ID NO: 25; and
FR4 consisting of the amino acid sequence represented by SEQ ID NO: 26.

In the present invention, the anti-PD-L1 hsdAb binds to an epitope of PD-L1, and the anti-CD47 hsdAb binds to an epitope of CD47.

In addition, the K_{D} for the binding of the anti-PD-L1×CD47 bsAb to PD-L1 and CD47, respectively, can range from 10⁻⁶ M to 10⁻¹² M, 10⁻⁶ M to 10⁻¹¹ M, 10⁻⁶ M to 10⁻¹⁰ M, 10⁻⁶ M to 10⁻⁹ M, 10⁻⁷ M to 10⁻⁹ M, or 10⁻⁸ M to 10⁻⁹ M

In addition, the EC₅₀ of the humanized anti-PD-L1×CD47 bsAb in FACS analysis may be less than 500 nM, and specifically may range from 0.01 nM to 500 nM, 0.01 nM to 400 nM, 0.01 nM to 300 nM, 0.01 nM to 200 nM, 0.01 nM to 100 nM, 0.01 nM to 50 nM, 0.01 nM to 10 nM, 0.1 nM to 500 nM, 0.1 nM to 400 nM, 0.1 nM to 300 nM, 0.1 nM to 200 nM, 0.1 nM to 100 nM, 0.1 nM to 50 nM, 0.1 nM to 10 nM, 1 nM to 500 nM, 1 nM to 400 nM, 1 nM to 300 nM, 1 nM to 200 nM, 1 nM to 100 nM, 1 nM to 50 nM, or 1 nM to 10 nM.

In the present invention, the humanized anti-PD-L1×CD47 bsAb may have any suitable valency for the epitopes of PD-L1 and CD47, respectively. Specifically, the humanized anti-PD-L1×CD47 bsAb may have a valency of 2, 3, 4, 5, 6, or higher for each of PD-L1 and CD47. For example, see P. Chames and D. Baty, Chapter 6. Bispecific Single Domain Antibodies, Springer-Verlag Berlin Heidelberg, 2011.

Additionally, the humanized anti-PD-L1×CD47 bsAb may be formed by the direct fusion of the anti-PD-L1 hsdAb and the anti-CD47 hsdAb through peptide bonding or by indirect fusion via a peptide linker. The length, flexibility, and/or other characteristics of the peptide linker may influence certain properties, including but not limited to affinity, specificity, or binding capacity, with respect to one or more specific antigens or epitopes. For example, a longer peptide linker may be selected to ensure that the two adjacent domains do not sterically interfere with each other. In certain embodiments, the peptide linker includes flexible residues (e.g., glycine and serine) to allow the adjacent domains to move freely relative to each other. For example, a glycine-serine doublet may be a suitable peptide linker. Additionally, the peptide linker may be of any suitable length, for instance, at least about any one of 1, 2, 3, 4, 5, 6, 7, 8, 9, 10, 11, 12, 13, 14, 15, 16, 17, 18, 19, 20, 25, 30, 35, 40, 50, 75, 100 or more amino acid residues in length. In some embodiments, the peptide linker may be a peptide linker consisting of any one of the amino acid sequences of SEQ ID NO: 27 to SEQ ID NO: 29.

Additionally, the peptide linker may have a naturally occurring sequence or a non-naturally occurring sequence.

In some embodiments, the humanized anti-PD-L1×CD47 bsAb may be bivalent for PD-L1 and monovalent for CD27, wherein the bivalent anti-PD-L1 hsdAb can be directly or indirectly fused to a monovalent anti-PD-L1 hsdAb, which is represented by the amino acid sequence SEQ ID NO: 16, either directly or via a peptide linker. Furthermore, the humanized anti-PD-L1×CD47 bsAb may consist of the amino acid sequence represented by SEQ ID NO: 15.

The single-domain antibody (sdAb) according to the present invention may be derived from any species, including but not limited to mouse, rat, human, camel, llama, cartilaginous fish, fish, shark, goat, rabbit, and bovidae. The naturally occurring sdAb molecules may also be included from species other than the Camelidae.

Additionally, the sdAb is a heavy-chain antibody lacking a light chain, derived from known naturally occurring single-domain antigen-binding molecules. Such single-domain molecules are disclosed, for example, in WO 94/04678 and Hamers-Casterman et al. (1993), Nature 363:446-448. The variable domains derived from heavy-chain molecules naturally lacking light chains are disclosed herein as VHH, distinguishing them from the conventional VH of the four-chain immunoglobulin. Such VHH molecules can be derived from antibodies produced by camelid species, including camels, llamas, vicugnas, dromedaries, alpacas, and guanacos. Other species, beyond the camelid family, that can produce heavy-chain molecules naturally lacking a light chain are also encompassed within the scope of the present invention.

According to the present invention, the humanized single domain antibody (hsdAb) is typically a humanized antibody in which a non-human antibody is humanized to reduce immunogenicity in humans while retaining the specificity and affinity of the parental non-human antibody. Generally, a humanized antibody comprises one or more variable domains in which the hypervariable regions (HVR), such as the complementarity-determining regions (CDR), or portions thereof, are derived from a non-human antibody, and the framework regions (FR), or portions thereof, are derived from a human antibody sequence. In some embodiments, the humanized single domain antibody comprises a CDR derived from a non-human single domain antibody **(e.g.,** a CDR derived from camelid species, such as llama) and a heavy chain FR derived from a human antibody sequence. Additionally, in some embodiments, certain FR residues in the humanized antibody are substituted with corresponding residues from a non-human antibody (e.g., the antibody from which the HVR residues are derived) to restore or enhance antibody specificity or affinity.

In the present invention, the bispecific antibody that binds bispecifically to PD-L1 and CD47, comprising the humanized anti-PD-L1×CD47 bsAb, may be a humanized anti-PD-L1×CD47 HCAb or an antigen-binding fragment thereof.

Specifically, the humanized anti-PD-L1×CD47 HCAb may be the humanized anti-PD-L1×CD47 bsAb described herein fused to one or more CH2 and/or CH3 domains, such as an Fc fragment. Furthermore, the anti-PD-L1 hsdAb and/or anti-CD47 hsdAb described herein may be fused to one or more CH2 and/or CH3 domains, such as an Fc fragment.

The CH2 and/or CH3 domain is derived from an immunoglobulin, which may be IgA, IgD, IgE, IgG, or IgM, and specifically may be IgG. In some embodiments, the humanized anti-PD-L1×CD47 HCAb may comprise an Fc fragment of IgG, for example, IgG1, IgG2, IgG3, or IgG4, wherein the Fc fragment may be a human Fc, such as a human IgG1 (hIgG1) Fc, hIgG2 Fc, hIgG3 Fc, or hIgG4 Fc.

The humanized anti-PD-L1×CD47 HCAb may be monomeric or multimeric. Furthermore, when multimeric, it may be bispecific and multivalent (e.g., bivalent, trivalent, tetravalent, or of higher valency) and may comprise two or more copies of the anti-PD-L1 hsdAb and anti-CD47 hsdAb described herein.

In the present invention, the humanized anti-PD-L1×CD47 bsAb, anti-PD-L1 hsdAb, or anti-CD47 hsdAb may be fused to a CH2 and/or CH3 domain, specifically an Fc fragment, through a peptide linker. The length, flexibility, and/or other characteristics of the peptide linker may influence certain properties, including but not limited to affinity, specificity, or binding capacity to one or more specific antigens or epitopes. For example, a longer peptide linker may be selected to ensure that two adjacent domains do not sterically hinder each other. In some embodiments, the peptide linker may include flexible residues (e.g., glycine and serine) to allow adjacent domains to move freely relative to each other. For instance, a glycine-serine doublet may be an appropriate peptide linker. Additionally, the peptide linker may be of any suitable length, such as at least about 1, 2, 3, 4, 5, 6, 7, 8, 9, 10, 11, 12, 13, 14, 15, 16, 17, 18, 19, 20, 25, 30, 35, 40, 50, 75, 100 amino acids, or more.

Additionally, the peptide linker may have a naturally occurring sequence or a non-naturally occurring sequence. For example, a sequence derived from the hinge region of a heavy chain-only antibody may be used as the linker. For instance, see WO 1996/34103. In some embodiments, the peptide linker may be a hIgG1 hinge, hIgG2 hinge, hIgG3 hinge, hIgG4 hinge, or variants thereof. Alternatively, it may be a peptide linker consisting of the amino acid sequence of SEQ ID NO: 30.

In the present invention, the humanized anti-PD-L1×CD47 HCAb may comprise the amino acid sequence represented by SEQ ID NO: 18, or a variant thereof that exhibits at least 80% sequence identity (e.g., at least about 80%, 88%, 90%, 91%, 92%, 93%, 94%, 95%, 96%, 97%, 98%, or 99%) to the amino acid sequence.

In the present invention, the bispecific antibody that binds bispecifically to PD-L1 and CD47, comprising the humanized anti-PD-L1×CD47 bsAb, may be a bispecific Fab-like antibody fragment (bsFab) in which the C-terminus of the anti-PD-L1 hsdAb and/or anti-CD47 hsdAb is fused to the N-terminus of the CH1 and Cκ domains.

The CH1 and Cκ domains are derived from an immunoglobulin. The immunoglobulin may be IgA, IgD, IgE, IgG, or IgM, and specifically may be IgG. The IgG may be IgG1, IgG2, IgG3, or IgG4, and may be human IgG1, IgG2, IgG3, or IgG4.

The bsFab and its manufacturing techniques according to the present invention are referenced from P. Chames and D. Baty, Chapter 6. Bispecific Single Domain Antibodies, Springer-Verlag Berlin Heidelberg, 2011.

In the present invention, the bispecific antibody that binds bispecifically to PD-L1 and CD47, comprising the humanized anti-PD-L1×CD47 bsAb, comprises amino acid sequence variants. The amino acid sequence variants of the antibody may be produced by introducing appropriate modifications into the nucleic acid sequence encoding the antibody or by peptide synthesis. Such modifications may include, for example, deletions, and/or insertions and/or substitutions of residues within the amino acid sequence of the antibody. Any combination of deletions, insertions, and substitutions may be made to yield the final product, with the understanding that the final product retains the desired characteristics, such as antigen-binding. In some embodiments, substitutions, insertions, or deletions may occur within one or more hypervariable regions (HVRs), provided that such modifications do not substantially reduce the antibody's ability to bind the antigen. For example, conservative changes that do not substantially reduce binding affinity may be made within the HVR. Such modifications may occur outside of the HVR "hotspot" or the CDR.

Additionally, the amino acid substitution may be at least one substitution (e.g., any of 1, 2, 3, 4, 5, 6, 7, 8, 9, or 10 amino acid substitutions). Furthermore, the at least one amino acid substitution may be a conservative substitution and may involve substitution with a non-genetically encoded amino acid or a synthetic amino acid. In some embodiments, the amino acid substitution may occur within the CDR regions, and may include at least one substitution (e.g., any of 1, 2, 3, or 4 amino acid substitutions) in CDR1, CDR2, and/or CDR3. In some embodiments, the amino acid substitution may occur within the FR regions, and may include at least one substitution (e.g., any of 1, 2, 3, 4, 5, or 6 amino acid substitutions) in FR1, FR2, FR3, and/or FR4.

Additionally, such amino acid sequence insertions include amino- and/or carboxyl-terminal fusions ranging in length from one residue to polypeptides containing more than 100 residues, as well as intra-sequence insertions of single or multiple amino acid residues. Examples of terminal insertions include antibodies with an N-terminal methionyl residue. Other insertion variants of the antibody molecule may include fusions to the N- or C-terminus of the antibody to polypeptides or (e.g., in the case of ADEPT) enzymes that increase the serum half-life of the antibody.

Furthermore, one or more amino acid modifications may be introduced into the Fc region of the bispecific antibody (e.g., the humanized anti-PD-L1×CD47 HCAb) that binds bispecifically to PD-L1 and CD47, as provided herein, thereby generating Fc region variants. The Fc region variant may include amino acid modifications (e.g., substitutions) at one or more amino acid positions in a human Fc region sequence (e.g., human IgG1, IgG2, IgG3, or IgG4 Fc).

In the present invention, the bispecific antibody that binds bispecifically to PD-L1 and CD47, comprising the humanized anti-PD-L1×CD47 bsAb, may be linked to, fused to, conjugated to (e.g., covalently or non-covalently), or otherwise associated with a diagnostic moiety or a biocompatibility modulator. For example, a peptide or polypeptide (e.g., biologic toxins, biomarkers, purification tags, etc.), protein, polymer, nucleic acid molecule, small molecule, mimetic, synthetic drug, inorganic molecule, organic molecule, or radioisotope may be conjugated or associated therewith.

Furthermore, the bispecific antibody that binds bispecifically to PD-L1 and CD47, comprising the humanized anti-PD-L1×CD47 bsAb, may be conjugated or associated with a diagnostic agent or detectable agent, marker, or reporter, which may be a biological molecule (e.g., peptide or nucleotide), small molecule, fluorophore, or radioisotope. The labeled modulators may be useful in monitoring diseases associated with PD-L1 and/or CD47, such as the onset or progression of cancer, or in determining the efficacy of a specific therapy that includes the antibody disclosed herein (i.e., theragnosis), or as part of clinical trial procedures to determine future treatment regimens. These markers or reporters may also be useful in purifying the antibodies disclosed herein.

Furthermore, the bispecific antibody that binds bispecifically to PD-L1 and CD47, comprising the humanized anti-PD-L1×CD47 bsAb, may be conjugated to an immunomodulator, cytokine, cytotoxic agent, chemotherapy agent, diagnostic agent, antiviral agent, antimicrobial agent, or drug. In this regard, the present invention provides an antibody conjugate comprising a bispecific antibody that binds bispecifically to PD-L1 and CD47, including the humanized anti-PD-L1×CD47 bsAb of the present invention conjugated to an immunomodulator, cytokine, cytotoxic agent, chemotherapeutic agent, diagnostic agent, antiviral agent, antimicrobial agent, or drug.

Additionally, the present invention provides a nucleic acid molecule encoding a bispecific antibody that binds bispecifically to PD-L1 and CD47, comprising the humanized anti-PD-L1×CD47 bsAb disclosed herein, an expression vector comprising the nucleic acid molecule, and a host cell transformed with the expression vector.

Additionally, the present invention provides a method for producing a bispecific antibody comprising the steps of:
(a) culturing the host cell under conditions that allow for the expression of the bispecific antibody; and
(b) recovering the expressed bispecific antibody.

In the present invention, the DNA encoding a bispecific antibody that binds bispecifically to PD-L1 and CD47, comprising the humanized anti-PD-L1×CD47 bsAb disclosed herein, can be easily isolated and sequenced using conventional procedures (e.g., by using oligonucleotide probes that can specifically bind to the genes encoding the antibody heavy and light chains). The isolated and subcloned hybridoma cells (or phage or yeast-derived colonies) may serve as a preferred source of DNA. More specifically, the isolated DNA (which may be modified) can be used to clone the constant and variable region sequences of the antibody for its production.

An exemplary method involves the extraction of RNA from the selected cells, conversion to cDNA, and amplification by PCR using antibody-specific primers. Suitable primers are well known in the art and, as exemplified herein, are readily available from numerous commercial sources. To express recombinant human or non-human antibodies isolated through screening of a combinatorial library, the DNA encoding the antibody is cloned into a recombinant expression vector and introduced into host cells, including mammalian cells, insect cells, plant cells, yeast, and bacteria. In some embodiments, the modulator is introduced into and expressed in cells such as monkey COS cells, NS0 cells, Chinese hamster ovary (CHO) cells, or myeloma cells that do not otherwise produce the desired construct.

In the present invention, the nucleic acid molecule is present within a vector, which, when appropriate, includes a promoter to control the expression of the nucleic acid. The vector is used in its most general sense and includes any intermediate vehicle for the nucleic acid that allows for the introduction of the nucleic acid into prokaryotic and/or eukaryotic cells, and, where appropriate, integration into the genome. Such vectors are preferably capable of replication and/or expression within the cells. The vector may include a plasmid, phagemid, bacteriophage, or viral genome. The plasmid is generally an extrachromosomal genetic construct that can replicate independently of chromosomal DNA, typically in the form of a circular DNA duplex.

An expression vector containing the antibody coding sequence and appropriate transcription and translation control signals can be constructed using methods widely known to those skilled in the art. These methods include, for example, in vitro recombinant DNA technology, synthetic techniques, and in vivo gene recombination.

In the present invention, the host cell or recombinant host cell refers to a cell into which an expression vector has been introduced. The recombinant host cell and host cell also refer to not only the specific target cell but also its descendants. Since specific modifications may arise in subsequent generations due to mutations or environmental influences, these descendants may not be identical to the parent cell but are still within the scope of the term "host cell" as used herein. Such cells may contain the vector described above.

Furthermore, using molecular biology techniques recognized in the art and current protein expression methodologies, a substantial quantity of the antibody disclosed herein can be produced. More specifically, the nucleic acid molecule encoding the antibody is integrated into widely known and commercially available protein production systems, including various types of host cells, to provide the desired pharmaceutical product in preclinical, clinical, or commercial quantities. In some embodiments, the nucleic acid molecule encoding the antibody is manipulated into a vector or expression vector that enables efficient integration into the selected host cell and provides high levels of subsequent antibody expression.

Preferably, the nucleic acid molecules encoding the antibody disclosed herein and the vectors comprising these nucleic acid molecules can be used for the transformation of suitable mammalian, plant, bacterial, or yeast host cells, although prokaryotic systems may also be used. Transformation can be carried out by any known method for introducing polynucleotides into host cells. Methods for introducing heterologous polynucleotides into mammalian cells are well known in the art and include techniques such as dextran-mediated transformation, calcium phosphate precipitation, polybrene-mediated transformation, protoplast fusion, electroporation, encapsulation of polynucleotides in liposomes, and direct microinjection of DNA into the nucleus. Additionally, the nucleic acid molecules can be introduced into mammalian cells using a viral vector. Methods for transforming mammalian cells are well known in the art. Methods for transforming plant cells are also widely recognized in the art, including, for example, Agrobacterium-mediated transformation, biolistic transformation, direct injection, electroporation, and viral transformation. Methods for transforming bacterial and yeast cells are also well known in the art.

A variety of commercially available host-expression vector systems can be used to express the antibody disclosed herein. These host-expression systems not only represent vehicles in which the coding sequence of interest is expressed and subsequently purified, but also represent cells capable of in vivo expression of the molecules of the present invention when transformed or transfected with the appropriate nucleotides coding sequence. These systems comprises microorganisms transformed with recombinant bacteriophage DNA, plasmid DNA, or cosmid DNA expression vectors containing modulator coding sequences, such as bacteria (e.g., E. coli, B. subtilis, streptomyces); yeast transfected with recombinant yeast expression vectors containing modulator coding sequences (e.g., Saccharomyces, Pichia); insect cell systems infected with recombinant virus expression vectors containing modulator coding sequences (e.g., baculovirus); plant cell systems that are infected with recombinant virus expression vectors (e.g., cauliflower mosaic virus, CaMV; tobacco mosaic virus, TMV) or transfected with recombinant plasmid expression vectors (e.g., Ti plasmid) containing modulator coding sequences (e.g., *Nicotiana, Arabidopsis,* duckweed, corn, wheat, potato, etc.); or mammalian cell systems containing recombinant expression constructs with promoters derived from mammalian genomes (e.g., metallothionein promoter) or from mammalian viruses (e.g., adenovirus late promoter; vaccinia virus 7.5K promoter) (e.g., COS, CHO, BHK, 293, 3T3 cells), but are not limited to these examples.

If the antibody disclosed herein is produced by recombinant expression or any other technique disclosed herein, it can be purified by any method known in the art for purifying immunoglobulins, or more generally, by any other standard techniques for protein purification.

The present invention also provides a pharmaceutical composition for the prevention or treatment of cancer, comprising as an active ingredient a bispecific antibody that binds bispecifically to PD-L1 and CD47, including the humanized anti-PD-L1×CD47 bsAb disclosed herein, or an antibody conjugate comprising said bispecific antibody.

The present invention also provides a method for the prevention or treatment of cancer, comprising the step of administering to an individual a pharmaceutical composition containing an effective amount of a bispecific antibody that binds bispecifically to PD-L1 and CD47, including the humanized anti-PD-L1×CD47 bsAb disclosed herein, or an antibody conjugate comprising said bispecific antibody.

Additionally, the present invention also provides the use of a bispecific antibody that binds bispecifically to PD-L1 and CD47, including the humanized anti-PD-L1×CD47 bsAb disclosed herein, or an antibody conjugate comprising said bispecific antibody, for the prevention or treatment of cancer.

The present invention further provides the use of a bispecific antibody that binds bispecifically to PD-L1 and CD47, including the humanized anti-PD-L1×CD47 bsAb disclosed herein, or an antibody conjugate comprising said bispecific antibody, for the manufacture of a composition for the prevention or treatment of cancer.

In the present invention, the cancer is one that requires activation of T cells by blocking the activity of immune checkpoint proteins, such as melanoma, lung cancer, liver cancer, glioblastoma, ovarian cancer, colorectal cancer, head and neck cancer, bladder cancer, renal cell carcinoma, gastric cancer, breast cancer, metastatic cancer, prostate cancer, pancreatic cancer, non-Hodgkin lymphoma, Hodgkin lymphoma, multiple myeloma, leukemia, lymphoma, myelodysplastic syndrome, acute lymphoblastic leukemia, acute myeloid leukemia, chronic lymphocytic leukemia, chronic myeloid leukemia, solitary plasmacytoma, and aplastic anemia, but is not limited thereto.

In the present invention, the content regarding the bispecific antibody that binds bispecifically to PD-L1 and CD47, including the humanized anti-PD-L1×CD47 bsAb disclosed herein, or the antibody conjugate comprising said bispecific antibody, is identical to the description provided above. Therefore, the specific explanation thereof is incorporated by reference, and hereinafter, only the unique configuration of the pharmaceutical composition and its uses will be described.

The pharmaceutical composition according to the present invention may comprise one or more (e.g., 2 or 3 types) of bispecific antibodies that bind bispecifically to PD-L1 and CD47, including the humanized anti-PD-L1×CD47 bsAb disclosed herein, or antibody conjugates comprising said bispecific antibodies.

By administering the pharmaceutical composition according to the present invention to an individual, specifically a cancer patient, cancer can be prevented or treated.

Furthermore, the pharmaceutical composition according to the present invention can be formulated as desired using techniques recognized in the relevant art, depending on the form of the antibody described herein, the intended delivery method, and various other factors. It may also be formulated to include suitable pharmaceutically acceptable carriers containing relatively inert substances, such as excipients and adjuvants, which are well-known in the art and assist in processing the active compound into a formulation that facilitates administration or is pharmaceutically optimized for delivery. For instance, various pharmaceutically acceptable carriers, including vehicles, adjuvants, and diluents, are readily available from numerous commercial sources. Additionally, classifications of pharmaceutically acceptable auxiliary agents, such as pH adjusters and buffers, tonicity adjusters, stabilizers, and wetting agents, are also available. Specific, non-limiting examples of such carriers include saline, buffered saline, dextrose, water, glycerol, ethanol, and combinations thereof.

Furthermore, the pharmaceutical composition of the present invention can be formulated for enteral, parenteral, or topical administration. In fact, all three types of formulations can be used simultaneously to achieve systemic administration of the active ingredient. Excipients and formulations for parenteral and non-parenteral drug delivery are known in the related art. Formulations suitable for parenteral administration include aqueous solutions of the active compound in a water-soluble form, such as aqueous solutions of water soluble salts. Additionally, suspensions of the appropriate active compound in an oily injection suspension may also be administered. Suitable lipophilic solvents or vehicles include fatty oils, such as sesame oil, or synthetic fatty acid esters, such as ethyl oleate or triglycerides. Aqueous injection suspensions may contain agents that increase the viscosity of the suspension, for example, sodium carboxymethylcellulose, sorbitol, and/or dextran. Optionally, the suspension may also contain stabilizers. Additionally, liposomes may be used to encapsulate the active agent for delivery into cells.

Formulations suitable for enteral administration include hard or soft gelatin capsules, pills, tablets including coated tablets, elixirs, suspensions, syrups, or inhalants, and controlled-release forms thereof.

In general, the antibodies disclosed herein may be administered in vivo to a subject in need thereof through various routes, including but not limited to oral, intravenous, intraarterial, subcutaneous, parenteral, intranasal, intramuscular, intracardiac, intraventricular, endotracheal, buccal, rectal, intraperitoneal, intradermal, topical, transdermal, and intrathecal administration, or via implantation or inhalation. Appropriate formulations and routes of administration may be selected depending on the intended use and therapeutic regimen.

The pharmaceutical composition according to the present invention is administered in a pharmaceutically effective amount for the treatment or prevention of cancer. The pharmaceutically effective amount refers to the amount of the antibody or pharmaceutical composition containing the antibody that is required to elicit a biological or medical response in the subject, as sought by a physician or other clinician. Additionally, multiple doses of the antibody or pharmaceutical composition containing the same may be administered at a specific frequency to achieve an amount of therapy having a preventive and/or therapeutic effect.

The pharmaceutically effective amount is typically dependent on factors such as the weight of the subject to be treated, the physical condition of the subject, the extent of the condition to be treated, and the age of the subject. Generally, the antibodies disclosed herein are administered in doses ranging from about 10 ng/kg body weight to about 100 mg/kg body weight, from about 50 µg/kg body weight to about 5 mg/kg body weight, from about 100 µg/kg body weight to about 10 mg per dose. /kg body weight range, from about 100 µg/kg body weight to about 20 mg/kg body weight, from 0.5 mg/kg body weight to about 20 mg/kg body weight. However, it is not limited to this. Additionally, the antibody may be administered in a dose of at least approximately 100 µg/kg body weight, at least approximately 250 µg/kg body weight, at least approximately 750 µg/kg body weight, at least approximately 3 mg/kg body weight, at least approximately 5 mg/kg body weight, or at least approximately 10 mg/kg body weight, but it is not limited to this.

Additionally, the pharmaceutical composition of the present invention may be administered in a dose ranging from approximately 100 mg to approximately 10,000 mg, approximately 200 mg to approximately 9,000 mg, approximately 300 mg to approximately 8,000 mg, approximately 400 mg to approximately 7,000 mg, or approximately 500 mg to approximately 5,000 mg, but this is not intended to be limiting.

The pharmaceutical composition according to the present invention is usually administered to a patient multiple times. Exemplary treatment regimens involve administration once every two weeks, once a month, or once every three to six months. For example, a patient may receive the antibody (e.g., as an intravenous formulation) once every four weeks in cycles, e.g., once every 28 days. The frequency of administration can be adjusted depending on the pharmacokinetic profile of the antibody in the patient. For example, the half-life of an antibody may require a two-week dosing frequency. In some methods, two or more antibodies with different binding specificities may be administered simultaneously, in which case the dosage of each antibody administered falls within the ranges indicated.

The dosage and frequency of administration are determined by the half-life of the antibody in the patient. Generally, human antibodies exhibit the longest half-life, followed by humanized antibodies, chimeric antibodies, and non-human antibodies. The dosage and administration frequency may vary depending on whether the treatment is prophylactic or therapeutic

The duration of the treatment regimen is influenced by factors such as the disease being treated, the patient's age and condition, the stage and type of the patient's disease, and how the patient responds to the treatment. The clinician may closely monitor the effectiveness of the therapy and make any necessary adjustments as needed. In cases where the active agent is used in combination, two or more therapeutic agents may be administered either simultaneously or in any sequential order, meaning that the antibody disclosed herein may be administered before, together with, or following the administration of the second therapeutic agent.

The present invention will be described in detail below with reference to embodiments and experimental examples.

However, it should be understood that the following embodiments and experimental examples are merely illustrative and are not intended to limit the scope of the present invention to the contents thereof.

### <Example 1> Immunization and Blood Collection

The immunization antigen, human PD-L1 protein or human CD47 protein, was mixed with an immunological adjuvant (GERBU) and administered via intramuscular injection to a single alpaca three times. Immunization was carried out in three rounds, and 14 days after the final immunization, blood samples of 10 mL each were collected from the alpaca. The immune response was analyzed using ELISA. To determine whether antibodies were produced, immunogenic antigens were dispensed at a concentration of 1 µg/ml into 96-well microplates using coating buffer and coated at 4°C overnight. The 96-well microplate was washed three times with PBST and then blocked by treating it with 5% skim milk for 2 hours at room temperature to inhibit non-specific binding. After washing three times with PBST, serum samples obtained before immunization (day 0), 14 days (day 14) after immunization, 28 days (day 28) after immunization, and 42 days (day 42) after immunization were processed at graded dilution concentrations. Afterwards, the 96-well microplate was washed five times with PBST, reacted with goat anti-Llama IgG HRP antibody at room temperature for 1 hour, and whether the antibody bound to the immunogenic antigen was confirmed through TMB reaction.

### <Example 2> Library Construction and Evaluation

An immune library was constructed through the amplification of genes encoding single domain antibodies that bind to the immunogenic antigen identified in <Example 1>. For library construction, peripheral blood mononuclear cells (PBMCs) were isolated from blood using Ficoll. A gene fragment encoding a single domain antibody was amplified from total RNA extracted from isolated peripheral blood mononuclear cells (PBMC) using specific primers, and cloned into pComb3x vector. The size of the produced immune library was 5.4 × 10⁸.

### <Example 3> Library Amplification

The immune library constructed in <Example 2> was transformed into XL1-Blue cells. The transformed XL1-Blue cells were added to 10 mL of 2x YT medium containing 2% glucose and 100 µg/mL ampicillin and cultured in a shaking incubator at 37°C. The culture was grown until an optical density at 600 nm (OD₆₀₀) of 0.5 was reached, at which point M13K07 phage (Invitrogen) was added to a final concentration of 1×10¹¹ pfu/mL. Subsequently, the culture was incubated statically at 37°C for 30 minutes, followed by an additional incubation in a shaking incubator at 37°C and 200 rpm for 30 minutes. The culture medium was then centrifuged at room temperature at 4,000 rpm for 15 minutes, and the supernatant was removed. Next, 10 mL of 2x YT medium containing 100 µg/mL ampicillin and 50 µg/mL kanamycin was added to resuspend the pellet, and the culture was incubated overnight in a shaking incubator at 30°C and 250 rpm. Following this, centrifugation was performed at 4°C and 4,000 rpm for 30 minutes. The supernatant was precipitated using the PEG precipitation method, followed by centrifugation at 4°C and 12,000 rpm for 30 minutes. The resulting pellet was resuspended in PBS and centrifuged at 4°C and 13,000 rpm for 5 minutes. The supernatant was transferred to a new tube and stored at 4°C until use.

### <Example 4> Bio-panning

To select single domain antibodies specific to the immunogenic antigen, the antigen was dispensed into a 96-well microplate at a concentration of 5 µg/mL using a coating buffer and was coated at 4°C overnight. The library to be used for the selection of single domain antibodies (the library of <Example 3>) was dispensed into a 96-well microplate and incubated at room temperature for 30 minutes. The library was then transferred to new wells and incubated at room temperature for an additional 30 minutes; this procedure was repeated four times to reduce nonspecific binding of the library to the microplate wells. The library was then transferred to a 1.7 mL tube and stored at 4°C until use. The microplate coated with the immunogenic antigen was washed five times with PBST and blocked with 5% skim milk at room temperature for 2 hours. After washing five times with PBST, the library, with reduced nonspecific binding, was dispensed with a binding solution (2.5% skim milk, 0.5% Tween 20) at a concentration of 5×10¹² virions/well and incubated at room temperature for 30 minutes. Subsequently, the plate was washed ten times with a washing solution (PBS, 0.5% Tween 20) followed by three additional washes with PBST. Single domain antibodies specifically bound to the immunogenic antigen were selectively eluted by adding 5 µg of the immunogenic antigen per well and incubating at room temperature with shaking at 500 rpm for 30 minutes. The eluted phages were then infected into exponentially growing XL1-Blue cells and plated on 2x YT agar medium. Panning for a second selection was repeated under the same conditions as described above. Single phage clones generated on the agar plates were each amplified and screened using FACS.

### <Example 5. Phage Screening

To induce transient overexpression of the immunogenic antigen in Expi-CHO cells, the gene encoding the immunogenic antigen was inserted into the pCMV6-GFP vector to construct the pCMV6-immunogenic antigen-GFP plasmid. Expi-CHO cells were washed with DPBS, followed by centrifugation at room temperature at 1,200 rpm for 3 minutes. The supernatant was removed, and the cells were resuspended in 2% skim milk and blocked at 4°C for 30 minutes. The cells were then centrifuged at room temperature at 1,200 rpm for 3 minutes, and the supernatant was removed. The cells were washed twice with DPBS and dispensed into a 96-well microplate at 3×10⁵ cells/100 µL/well. Single-clone phages were added to each well and incubated at 4°C for 1 hour, followed by two washes with DPBS. An Antibody (M13 major coat protein Alexa Fluor 647 (Santa Cruz)) that specifically binds to the phage were dispensed into the cells and incubated in the dark at 4°C for 30 minutes. The cells were washed twice with DPBS, resuspended in fresh DPBS, and analyzed by FACS using the Accuri C6 (BD) instrument. Clones screened using the FACS system were selected and subjected to sequence analysis.

### <Example 6> Expression and Purification of Single Domain Antibody Fused with Human IgG Fc Domain

### <Example 6-1> Expression and purification of single monovalent domain antibody fused with human IgG Fc domain

The clones selected in <Example 5> were cloned into the TGEX-Fc (IgG1) or TGEX-Fc (IgG4) expression vectors. For the expression of single domain antibodies fused with the human IgG Fc domain, Expi-CHO cells with a viability of 95-99% were counted and 7×10⁶ cells were added to 25 mL of culture medium (Expi-CHO expression medium, Gibco). The cells were cultured overnight in a shaking incubator at 37°C and 125 rpm with 8% CO₂. Subsequently, 80 µL of ExpiFectamine^{™} CHO Reagent (Gibco, 100033021) was mixed with 920 µL of OptiPRO^{™} medium, and 20 µg of plasmid DNA encoding the single domain antibody fused with the human IgG Fc domain was added, followed by 1 mL of OptiPRO^{™} medium. The mixture was incubated at room temperature for 5 minutes and then added to the cultured cells. The cells were cultured for 20 hours in a shaking incubator at 125 rpm and 8% CO₂. To enhance the expression of the single domain antibody fused with the human IgG Fc domain, 150 µL of ExpiFectamine^{™} CHO enhancer (Gibco) and 6 mL of ExpiCHO Feed (Gibco) were added, and the culture was continued for 5 days in a shaking incubator at 125 rpm and 5% CO₂ at 32°C. The cultured cells were then centrifuged at 4°C and 4,000 rpm for 30 minutes, and the supernatant was filtered through a 0.2 µm syringe filter. The supernatant was loaded onto a HiTrap Protein G HP column (GE Healthcare), washed with PBS, and the single domain antibody fused with the human IgG Fc domain was eluted from the column using IgG elution buffer (Thermo). The eluted sample was neutralized by adding 1M Tris-HCl (pH 9.0) and stored at 4°C until use.

### <6-2> Expression and purification of single bivalent domain antibody fused with human IgG Fc domain

The clones selected in <Example 5> were linked using two G2S linkers (GGSGGS) to construct a bivalent single domain antibody. The nucleotide sequence encoding the bivalent single domain antibody was obtained through gene synthesis (Macrogen, South Korea). For the expression and purification of the single domain antibody fused with the human IgG4 Fc domain, the synthesized gene was cloned into the TGEX-Fc (IgG4) expression vector. The expression and purification were then performed using the same method as described in <Example 6-1>.

### <Example 7> Expression and Purification of Bispecific Single Domain Antibody Fused with Human IgG4 Fc Domain

Among the clones selected in <Example 5>, one single domain antibody specific to PD-L1 and one specific to CD47 were selected. The nucleotide sequences encoding each single domain antibody were linked using a peptide linker, and two single domain antibodies specific to PD-L1 were linked in sequence with a single domain antibody specific to CD47 (anti-PD-L1 sdAb × anti-PD-L1 sdAb × anti-CD47 sdAb). The nucleotide sequence encoding this trivalent bispecific single domain antibody was obtained through gene synthesis (Macrogen, South Korea). For the expression and purification of the bispecific single domain antibody fused with the human IgG4 Fc domain, the synthesized gene was cloned into the TGEX-Fc (IgG4) expression vector, and expression and purification were performed using the same method as described in <Example 6-1>.

### <Example 8> Expression and Purification of Bispecific Humanized Single Domain Antibody Fused with Human IgG4 Fc Domain

To humanize the trivalent bispecific single domain antibody produced in <Example 7>, a comparison of the FR (framework region) sequence of the human germline VH segment DP-47 (J Mol Biol. 1992 Oct 5;227(3):776-98) was performed to assess homology (identity). Among the clones selected in <Example 5>, one single domain antibody specific to PD-L1 and one specific to CD47 (PDL1 Nb#01 and CD47 Nb#01) were analyzed for their amino acid sequences. The first amino acid in the FR1 (framework region) sequence, glutamine (Q), was substituted with glutamic acid (E) to improve homology with the human antibody DP-47. The resulting trivalent bispecific humanized single domain antibody (humanized anti-PD-L1 sdAb × humanized anti-PD-L1 sdAb × humanized anti-CD47 sdAb) was generated. The amino acid substitution was performed using the Q5 Site Directed Mutagenesis Kit (NEB) and specific primers for targeted mutagenesis. Expression and purification were performed using the same method as described in <Example 6>.

### <Example 9> Evaluation of Binding Affinity of Single Domain Antibodies to Immunogenic Antigens Using FACS

The binding affinity of anti-PD-L1 HCAb (PDL1 Nb#01-IgG1), anti-PD-L1 bivalent HCAb (PP Nb-IgG4), anti-CD47 HCAb (CD47 Nb-IgG4), anti-PD-L1×CD47 trivalent HCAb (PPC Nb-IgG4), and humanized anti-PD-L1×CD47 trivalent HCAb (hPPC Nb-IgG4), as purified in <Examples 6>, <7>, and <8>, to the immunogenic antigens was evaluated using FACS.

Specifically, the CHO-K1_PD-L1 cell line (CHO-K1 cells overexpressing the PD-L1 antigen) or Expi-CHO_CD47 cell line (Expi-CHO cells overexpressing the CD47 antigen) was washed with DPBS and then centrifuged at 1,200 rpm for 3 minutes at room temperature. The supernatant was removed, and the cells were resuspended in 2% skim milk and blocked at 4°C for 30 minutes. The cells were then centrifuged again at 1,200 rpm for 3 minutes at room temperature, the supernatant was discarded, and the cells were washed twice with DPBS. Then, the cells were distributed to each well at a density of 3×10⁵ cells/100 µL/well and anti-PD-L1 HCAb (PDL1 Nb#01-IgG1), anti-PD-L1 bivalent HCAb (PP Nb-IgG4), anti-CD47 HCAb (CD47 Nb-IgG4), anti-PD-L1×CD47 trivalent HCAb (PPC Nb-IgG4), or humanized anti-PD-L1×CD47 trivalent HCAb (hPPC Nb-IgG4) was treated at different concentrations. An isotype control antibody was used as a negative control. The cells were incubated at 4°C for 1 hour, followed by two washes with DPBS. Subsequently, the cells were treated with an antibody specific to the human Fc domain (Anti-human IgG Fc APC antibody (Biolegend)) and incubated at 4°C for 30 minutes in the dark. After two additional washes with DPBS, the cells were resuspended in 100 µL of DPBS and analyzed using FACS with an Accuri C6 (BD) system.

### <Example 10> Evaluation of the Inhibitory Effet of Single Domain Antibodies on an Immunogenic Antigen Interaction Using FACS

The inhibitory effect of anti-PD-L1 HCAb (PDL1 Nb#01-IgG1), anti-PD-L1 bivalent HCAb (PP Nb-IgG4), anti-CD47 HCAb (CD47 Nb-IgG4), anti-PD-L1×CD47 trivalent HCAb (PPC Nb-IgG4), and humanized anti-PD-L1×CD47 trivalent HCAb (hPPC Nb-IgG4), as purified in <Examples 6>, <7>, and <8>, on the PD-1/PD-L1 or CD47/SIRPalphainteractions, was evaluated.

Specifically, to evaluate the interaction of anti-PD-L1 HCAb (PDL1 Nb#01-IgG1), anti-PD-L1 bivalent HCAb (PP Nb-IgG4), anti-PD-L1×CD47 trivalent HCAb (PPC Nb-IgG4), or humanized anti-PD-L1×CD47 trivalent HCAb (hPPC Nb-IgG4) with the PD-1/PD-L1 interaction, CHO-K1_PD-L1 cell line (CHO-K1 cells stably expressing PD-L1 antigen) was seeded in a 96-well microplate at 2×10⁵ cells per well and treated with 10 µg/mL of human PD-1-His protein. Then, anti-PD-L1 HCAb (PDL1 Nb#01-IgG1), anti-PD-L1 bivalent HCAb (PP Nb-IgG4), anti-PD-L1×CD47 trivalent HCAb (PPC Nb-IgG4), or humanized anti-PD-L1×CD47 trivalent HCAb (hPPC Nb-IgG4) was treated at different concentrations. An isotype control antibody was used as a negative control. The cells were incubated at 4°C for 1 hour, followed by three washes with DPBS. Subsequently, an antibody specific for His antigen (Goat anti-His APC) was added, and the reaction was carried out at 4°C for 30 minutes in the dark. The cells were washed three times with DPBS and then resuspended in 100 µL of DPBS. The Amount of PD-1-His protein remaining in CHO-K1_PD-L1 cells (CHO-K1 cells stably expressing PD-L1 antigen) was confirmed using Accuri C6 (BD) equipment. The inhibitory effect of anti-PD-L1 HCAb (PDL1 Nb#01-IgG1), anti-PD-L1 bivalent HCAb (PP Nb-IgG4), anti-PD-L1×CD47 trivalent HCAb (PPC Nb-IgG4), or humanized anti-PD-L1×CD47 trivalent HCAb (hPPC Nb-IgG4) on the PD-1/PD-L1 interaction was evaluated by measuring the remaining PD-1-His protein.

To evaluate the effect of anti-CD47 sdAb (CD47 Nb-IgG4), anti-PD-L1×CD47 trivalent HCAb (PPC Nb-IgG4), or humanized anti-PD-L1×CD47 trivalent HCAb (hPPC Nb-IgG4) on the CD47/SIRPalpha interaction, Expi-CHO_CD47 cells (Expi-CHO cells stably expressing CD47 antigen) were seeded in a 96-well microplate at 2×10⁵ cells per well and treated with 10 µg/mL human SIRPalpha-His protein. Then, anti-CD47 sdAb (CD47 Nb-IgG4), anti-PD-L1×CD47 3-valent HCAb (PPC Nb-IgG4), or humanized anti-PD-L1×CD47 3-valent HCAb (hPPC Nb-IgG4) was treated at different concentrations. An isotype control antibody was used as a negative control. Next, the amount of SIRPalpha-His protein remaining in Expi-CHO_CD47 cells (Expi-CHO cells stably expressing CD47 antigen) was confirmed using Accuri C6 (BD) equipment in the same manner as described above. The inhibitory effect of anti-CD47 sdAb (CD47 Nb-IgG4), anti-PD-L1×CD47 HCAb (PPC Nb-IgG4), or humanized anti-PD-L1×CD47 HCAb (hPPC Nb-IgG4) on the CD47/SIRPalpha interaction was evaluated by measuring the remaining amount of SIRPα-His protein.

### <Example 11> Evaluation of affinity of single domain antibody with an immunogenic antigen

The affinity (K_{d}) of the anti-PD-L1 HCAb (PDL1 Nb#01-IgG1), anti-PD-L1 bivalent HCAb (PP Nb-IgG4), anti-CD47 HCAb (CD47 Nb-IgG4), anti-PD-L1×CD47 trivalent HCAb (PPC Nb-IgG4), or humanized anti-PD-L1×CD47 trivalent HCAb (hPPC Nb-IgG4), purified in <Examples 6>, <7>, and <8>, for the immunogenic antigen protein was measured using an Octet RED 96e (ForteBio) instrument.

Specifically, biosensor tips (Fortebio) coated with anti-human Fc were saturated with 5 µg/mL of anti-PD-L1 HCAb (PDL1 Nb#01-IgG1), anti-PD-L1 bivalent HCAb (PP Nb-IgG4), anti-CD47 HCAb (CD47 Nb-IgG4), anti-PD-L1×CD47 trivalent HCAb (PPC Nb-IgG4), or humanized anti-PD-L1×CD47 trivalent HCAb (hPPC Nb-IgG4) at the level of 1.5 nm in a 96-well microplate (Greiner), respectively. The PD-L1 and CD47 antigens were serially diluted twofold from 10 to 400 nM using 1X kinetic buffer (ForteBio) and reacted with the coated biosensors at 30°C and 1,000 rpm. The binding and dissociation reactions of the samples were analyzed over 200 and 400 seconds, respectively. The result data were analyzed using a 1:1 interaction model (Global fitting) method.

Furthermore, the affinity (K_{d}) of the humanized anti-PD-L1×CD47 trivalent HCAb (hPPC Nb-IgG4), as purified in <Example 8>, for cross-species immunogenic antigen proteins was measured using the Octet RED 96e instrument (ForteBio).

Specifically, biosensor tips (Fortebio) coated with anti-human Fc were saturated with 20 µg/mL of humanized anti-PD-L1×CD47 trivalent HCAb (hPPC Nb-IgG4) at the level of 1.5 nm in a 96-well microplate (Greiner). The PD-L1 or CD47 antigens from human, mouse, and cynomolgus monkey were serially diluted twofold from 12.5 to 200 nM using 1X kinetic buffer (ForteBio) and reacted with the coated biosensors at 30°C with agitation at 1,000 rpm. The binding and dissociation reactions of the samples were analyzed over 200 and 400 seconds, respectively. The result data were analyzed using a 1:1 interaction model (Global fitting) method.

### <Example 12> In vitro Efficacy Evaluation of Humanized Single Domain Antibody

The *in vitro* efficacy of the humanized anti-PD-L1×CD47 trivalent HCAb (hPPC Nb-IgG4), as purified in <Example 8>, was evaluated by assessing T cell activation, the degree of CD4⁺ T cell activation in a mixed lymphocyte reaction, and the extent of phagocytosis activation by macrophages.

### <12-1> Evaluation of T cell activity of humanized single domain antibody

The T cell activation efficacy of the humanized anti-PD-L1×CD47 trivalent HCAb (hPPC Nb-IgG4), as purified in <Example 8>, was evaluated by measuring the IL-2 concentration expressed by Jurkat cells. This was done by assessing the inhibition of interaction between CHO-K1 cells, which were induced to overexpress PD-L1 protein, and Jurkat cells expressing PD-1.

Specifically, CHO-K1 cells overexpressing PD-L1 protein were dispensed at 2×10⁴ cells per well and incubated for 16 hours in an incubator maintained at 5% CO₂. The medium was then removed, and humanized anti-PD-L1×CD47 trivalent HCAb (hPPC-Nb-IgG4) was added at various concentrations and reacted for 1 hour. Jurkat cells were prepared at 5×10⁵ cells/100 µL and treated with PHA at a final concentration of 0.5 mg/mL. These Jurkat cells were then added to the CHO-K1 cells pre-treated with humanized anti-PD-L1×CD47 trivalent HCAb (hPPC-Nb-IgG4) and incubated for 48 hours. The IL-2 concentration in the supernatant was subsequently measured by ELISA to evaluate the T cell activation efficacy of the humanized anti-PD-L1×CD47 trivalent HCAb (hPPC-Nb-IgG4).

### <12-2> Evaluation of CD4⁺ T cell activation in mixed lymphocyte reaction of humanized single domain antibody

The activation of CD4⁺ T cells in a mixed lymphocyte reaction (MLR) assay was evaluated for the humanized anti-PD-L1×CD47 trivalent HCAb (hPPC Nb-IgG4), as purified in <Example 8>.

Specifically, monocyte-derived dendritic cells (MoDCs) from Donor A were dispensed at 5×10⁴ cells/well in a 96-well flat-bottom plate, and CD4⁺ T cells from Donor B were added to each well containing MoDCs at 4×10⁵ cells/well, allowing them to mix. Humanized anti-PD-L1×CD47 trivalent HCAb (hPPC-Nb-IgG4) was then added at various concentrations, and the cells were incubated for 5 days (120 hours) in a 37°C incubator with 5% CO₂. On the third day of incubation, the supernatant was collected for IL-2 measurement, and on the fifth day, the supernatant was collected from the same wells for IFN-γ measurement. The concentrations of IL-2 and IFN-γ were then determined using ELISA.

The MoDCs used in the mixed lymphocyte reaction analysis express PD-L1, and the CD4⁺ T cells express PD-1. When the interaction between PD-L1 and PD-1 is disrupted by the antibody, the suppressed activity of T cells can be activated. This is similar to inhibiting the system in which cancer cells survive within the immune system by suppressing immune activity through the binding of PD-L1 expressed on the cancer cells and PD-1 expressed on T cells. Therefore, by performing the mixed lymphocyte reaction analysis and measuring the concentrations of IL-2 and IFN-γ, which are indicators of T cell activation, the efficacy of humanized anti-PD-L1×CD47 trivalent HCAb (hPPC Nb-IgG4) was evaluated.

### <12-3> The evaluation of phagocytosis activation by macrophages of the humanized single-domain antibody

Phagocytosis is activated by the antigen-specific binding and the IgG4 Fc domain. The antigen-specific binding is influenced by the physical (intercellular distance), chemical (chemokine), and biological (cytokine) environments between the target cell and the effector cell. The PD-L1 and CD47 antigen-specific humanized antibodies specifically bind to hPDL1 and hCD47 expressed on the target cells, and the Fc region binding site binds to the Fc receptors on the effector cell surface, thereby promoting the interaction between the target cell and effector cell, which induces phagocytosis by macrophages.

Thus, the activation of phagocytosis by macrophages was evaluated through the CD47 antigen-specific binding of the humanized anti-PD-L1×CD47 trivalent HCAb (hPPC Nb-IgG4), as purified in <Example 8>.

Specifically, 5×10⁶ cells of THP-1 cells were seeded into a 100 phi dish and cultured for 24 hours in a 5% CO₂ incubator. After 24 hours, the cells were treated with 40 nM PMA for 24 hours, and the medium was removed. The cells were then stained with 1 µM deep red dye. After staining, the cells were replaced with fresh medium and rested for 48 hours. The THP-1 cells, detached with trypsin, were mixed at an 8:1 ratio with prey cells (Raji cells) stained with 3 µM CFSE, and treated with humanized anti-PD-L1×CD47 trivalent HCAb (hPPC Nb-IgG4) at various concentrations. The cells were co-cultured for 4 hours. Phagocytosis was then evaluated using FACS.

### <12-4> Evaluation of phagocytosis activation by PBMC-derived macrophages of humanized single domain antibodies

Monocytes isolated from human PBMCs were differentiated into macrophages and co-cultured with target cells (Raji_PDL1) to evaluate the activation of phagocytosis by human monocyte-derived macrophages through the hPD-L1 and hCD47 antigen-specific binding of the humanized anti-PD-L1×CD47 trivalent HCAb (hPPC Nb-IgG4), as purified in <Example 8>.

Specifically, monocytes were isolated from PBMCs separated from whole blood through Ficoll gradient, using the CD14⁺ MACS kit, and differentiated into macrophages by treating them with 50 ng/ml M-CSF for 6 days at a concentration of 2×10⁶ cells/ml in 100 mm dishes. After differentiation, the macrophages were stained with 5 µM CFSE as effector cells, and the target cells (Raji_PDL1) were stained with 10 µM Deep Red dye. The target cells were plated at 2×10⁴ cells/well, and the effector-to-target cell ratio was 8:1 for co-culture. Additionally, humanized anti-PD-L1×CD47 trivalent HCAb (hPPC Nb-IgG4) was serially diluted from 1000 nM to 1/5 and evaluated at 15 concentration points. After 4 hours of co-culture, FACS analysis was performed to confirm the expression of Deep Red (APC) and CFSE (FITC), and based on CFSE expression, the APC-expressing cell population was evaluated to determine the degree of phagocytosis

### <Example 13> In vitro safety evaluation of humanized single-domain antibody

*In vitro* safety of the humanized anti-PD-L1×CD47 trivalent HCAb (hPPC Nb-IgG4), purified in <Example 8>, was evaluated by confirming human RBC binding and hemagglutination reaction.

### <13-1> Evaluation of RBC binding of humanized single domain antibody

Human RBC binding, which occurs when the humanized anti-PD-L1×CD47 trivalent HCAb (hPPC Nb-IgG4) purified in <Example 8> specifically binds to the CD47 antigen, was evaluated.

Specifically, to assess the binding ability of human RBC (Red Blood Cells) to humanized anti-PD-L1×CD47 trivalent HCAb (hPPC Nb-IgG4), RBCs were washed seven times with DPBS and then diluted to 12% (v/v) in DPBS. 50 µL of humanized anti-PD-L1×CD47 trivalent HCAb (hPPC Nb-IgG4) was added to a 96-well V-bottom plate, followed by the addition of 50 µL of 12% (v/v) RBCs, and the reaction was carried out at 4°C for 1 hour. After washing the cells with DPBS, the cells were treated with an anti-human IgG4 secondary antibody and incubated at 4°C for 1 hour. After a final wash with DPBS, the extent of binding of humanized anti-PD-L1×CD47 trivalent HCAb (hPPC Nb-IgG4) to RBCs was evaluated by FACS.

### <13-2> Evaluation of hemagglutination response by humanized single domain antibody

The hemagglutination response induced by the specific binding of the humanized anti-PD-L1×CD47 trivalent HCAb (hPPC Nb-IgG4), purified in <Example 8>, to the CD47 antigen was evaluated.

Specifically, to evaluate the hemagglutination response of the humanized anti-PD-L1×CD47 trivalent HCAb (hPPC Nb-IgG4), RBCs were washed seven times with DPBS and then diluted to 6% (v/v) with DPBS. 50 µL of 2X humanized anti-PD-L1×CD47 trivalent HCAb (hPPC Nb-IgG4) was added to a 96-well U-bottom plate, followed by the addition of 50 µL of 6% (v/v) RBCs. The reaction was allowed to proceed for 1 hour at room temperature, after which the hemagglutination response was observed visually.

### <Example 14> In vivo Efficacy Evaluation of Humanized Single-Domain Antibody

The in vivo efficacy of the humanized anti-PD-L1×CD47 trivalent HCAb (hPPC Nb-IgG4), purified in <Example 8>, was evaluated by assessing its antitumor effect in C57BL/6 mice and humanized NSG mice bearing tumors formed by the injection of tumor cell lines expressing human PD-L1 and human CD47.

### <14-1> C57BL/6 mouse model using B16F10_PD-L1_CD47 tumor

6-8 week-old C57BL/6 mice were injected with 8×10**⁵** cells/100 µL of B16F10 cell line overexpressing human PD-L1 and human CD47. Tumor formation was induced until the tumor reached a size of 3 × 3 mm in the length (long axis) × width (short axis) dimensions. Subsequently, humanized anti-PD-L1×CD47 trivalent HCAb (hPPC Nb-IgG4) at a dose of 10 mg/kg was administered intraperitoneally 7 times at 2-day intervals, and tumor size was measured. After the final intraperitoneal injection, tumor volume was measured at 2-day intervals for 2 weeks. Tumor volume was calculated using the formula: length (long axis) × width (short axis) × height (short axis) / 2.

### <14-2> Humanized NSG mouse model using Raji_PD-L1 tumor

6-8 week-old female NSG mice were intraperitoneally injected with 2×10⁷ cells/100 µL of human PBMCs to generate humanized NSG mice. Then, 2×10⁶ cells/100 µL of human Raji (PD-L1) lymphoma cell line, which induced overexpression of human PD-L1, were injected. Tumor formation was induced until the tumor reached a size of 3 × 3 mm (length × width). Subsequently, 10 mpk of humanized anti-PD-L1×CD47 trivalent HCAb (hPPC Nb-IgG4) was administered intraperitoneally three times a week, and tumor volume was measured. The experiment was terminated when the tumor volume reached 2000 mm³ after the final intraperitoneal dose.

### <14-3> Humanized NSG mouse model using MDA-MB-231 tumor

6-8 week-old female NOD scid NSG mice were intravenously injected with 2×10⁷ cells/100 µL of human PBMCs to generate humanized NSG mice. After confirming the expression of hCD45 and mCD45, 2×10⁶ cells/100 µL of human breast cancer cell line MDA-MB-231 were subcutaneously injected into the right flank. Tumor formation was induced until the tumor reached a size of 3 × 3 mm (length × width). Subsequently, humanized anti-PD-L1×CD47 trivalent HCAb (hPPC Nb-IgG4) at doses of 1, 5, and 10 mpk was administered intraperitoneally three times a week, and tumor volume was measured. Additionally, when the tumor volume of the humanized NSG mice reached 100 mm³, Kaplan-Meier survival analysis was performed to predict the survival rate based on the occurrence of animal mortality.

### <Experimenal Example 1> Production of Anti-PD-L1 HCAb (PDL1 Nb#01-IgG1) and in vitro Characterization

### <1-1> Production of anti-PD-L1 HCAb (PDL1 Nb#01-IgG1)

Using human PD-L1 antigen as an immunogenic antigen, a single domain antibody clone specific to PD-L1 antigen was selected using the same method as described in <Example 5>, and sequence analysis was performed. The amino acid sequence of the selected anti-PD-L1 sdAb (PDL1 Nb#01) is shown in [Table 5] and [Table 6] below.

Additionally, using the selected anti-PD-L1 sdAb (PDL1 Nb#01), a monovalent single domain antibody specific to PD-L1 containing the human IgG1 Fc domain was expressed and purified using the same method described in <Example 6-1>, and the purified monovalent single domain antibody was named anti-PD-L1 HCAb (PDL1 Nb#01-IgG1). The amino acid sequence of the anti-PD-L1 HCAb (PDL1 Nb#01-IgG1) containing the human IgG1 Fc domain is shown in [Table 7] below.

Additionally, using the selected anti-PD-L1 sdAb (PDL1 Nb#01) clone, a divalent single domain antibody specific to PD-L1 containing the human IgG4 Fc domain was expressed and purified using the same method described in <Example 6-2>, and the purified divalent single domain antibody was named anti-PD-L1 2-valent HCAb (PP Nb-IgG4). The amino acid sequence of the anti-PD-L1 2-valent sdAb (PP Nb), excluding the human IgG4 Fc domain, is shown in [Table 8] below, and the amino acid sequence of the anti-PD-L1 2-valent HCAb (PP Nb-IgG4) containing the human IgG4 Fc domain is shown in [Table 9] below.

**[Table 5]**

| Single Domain Antibody | Single Domain Antibody Sequence | Sequence Number |
|---|---|---|
| Anti-PD-L1 sdAb (PDL1 Nb#01) | QVQLVESGGGLVQPGGSLRLSC AASGFTFSSFAMSWVRQAPGKG LEWVSDINTGGDSTDYADSVKG RFTISRDNAKNTLYLQMNSLKP EDTAVYYCAKGPKEMVHVYSQR GQGTQVTVSS | 1 |

**[Table 6]**

| Single Domain Antibody | CDR1 | Sequence Number | CDR2 | Sequence Number | CDR3 | Sequence Number |
|---|---|---|---|---|---|---|
| Anti-PD-L1 sdAb (PDL1 Nb#01) | GFTFSSFA | 2 | INTGGDST | 3 | AKGPKEMVHVYSQ | 4 |

**[Table 7]**

| Single Domain Antibody | Single Domain Antibody Sequence + Human IgG1 Fc Sequence | Sequence Number |
|---|---|---|
| Anti-PD-L1 HCAb (PDL1 Nb#01-IgG1) | QVQLVESGGGLVQPGGSLRLSCAASGFTFSSFAMSWVRQAPG KGLEWVSDINTGGDSTDYADSVKGRFTISRDNAKNTLYLQMN SLKPEDTAVYYCAKGPKEMVHVYSQRGQGTQVTVSSGPGGPE PKSSDKTHTCPPCPAPELLGGPSVFLFPPKPKDTLMISRTPE VTCVVVDVSHEDPEVKFNWYVDGVEVHNAKTKPREEQYNSTY RVVSVLTVLHQDWLNGKEYKCKVSNKALPAPIEKTISKAKGQ PREPQVYTLPPSRDELTKNQVSLTCLVKGFYPSDIAVEWESN GQPENNYKTTPPVLDSDGSFFLYSKLTVDKSRWQQGNVFSCS VMHEALHNHYTQKSLSLSPGK | 5 |

**[Table 8]**

| Single Domain Antibody | Single Domain Antibody Sequence | Sequence Number |
|---|---|---|
| Anti-PD-L1 bivalent sdAb (PP Nb) | QVQLVESGGGLVQPGGSLRLSCAASGFTFSSFAMSWVRQAPG KGLEWVSDINTGGDSTDYADSVKGRFTISRDNAKNTLYLQMN SLKPEDTAVYYCAKGPKEMVHVYSQRGQGTQVTVSSGGSGGS QVQLVESGGGLVQPGGSLRLSCAASGFTFSSFAMSWVRQAPG KGLEWVSDINTGGDSTDYADSVKGRFTISRDNAKNTLYLQMN SLKPEDTAVYYCAKGPKEMVHVYSQRGQGTQVTVSS | 6 |

**[Table 9]**

| Single Domain Antibody | Single Domain Antibody Sequence + Human IgG4 Fc Sequence | Sequence Number |
|---|---|---|
| Anti-PD-L1 bivalent HCAb (PP Nb-IgG4) | QVQLVESGGGLVQPGGSLRLSCAASGFTFSSFAMSWVRQAPG KGLEWVSDINTGGDSTDYADSVKGRFTISRDNAKNTLYLQMN SLKPEDTAVYYCAKGPKEMVHVYSQRGQGTQVTVSSGGSGGS QVQLVESGGGLVQPGGSLRLSCAASGFTFSSFAMSWVRQAPG KGLEWVSDINTGGDSTDYADSVKGRFTISRDNAKNTLYLQMN SLKPEDTAVYYCAKGPKEMVHVYSQRGQGTQVTVSSGPGGPE SKYGPPCPPCPAPEFLGGPSVFLFPPKPKDTLMISRTPEVTC VVVDVSQEDPEVQFNWYVDGVEVHNAKTKPREEQFNSTYRVV | 7 |
| | SVLTVLHQDWLNGKEYKCKVSNKGLPSSIEKTISKAKGQPRE PQVYTLPPSQEEMTKNQVSLTCLVKGFYPSDIAVEWESNGQP ENNYKTTPPVLDSDGSFFLYSRLTVDKSRWQEGNVFSCSVMH EALHNHYTQKSLSLSPGK | |

### <1-2> Evaluation of antigen binding affinity and inhibitory effect on PD-1/PD-L1 interaction of anti-PD-L1 HCAb (PDL1 Nb#01-IgG1) and anti-PD-L1 2-valent HCAb (PP Nb-IgG4)

Using the method described in <Example 9> and <Example 10>, the antigen binding affinity (Fig. 1a and Fig. 2a) and the inhibitory effect on the PD-1/PD-L1 interaction (Fig. 1b and Fig. 2b) of the anti-PD-L1 HCAb (PDL1 Nb#01-IgG1) and anti-PD-L1 2-valent HCAb (PP Nb-IgG4) purified in <Experiment 1> were evaluated by FACS.

As a result, as shown in Fig. 1a and Fig. 1b, the anti-PD-L1 HCAb (PDL1 Nb#01-IgG1) exhibited an antigen binding affinity of 23.31 nM (EC50) in CHO-K1_PD-L1 cells (CHO-K1 cells stably expressing PD-L1 antigen) and an inhibitory effect on the PD-1/PD-L1 interaction with an IC50 of 4.60 nM.

Additionally, as shown in Fig. 2a and Fig. 2b, the anti-PD-L1 2-valent HCAb (PP Nb-IgG4) exhibited an antigen binding affinity of 1.93 nM (EC50) in CHO-K1_PD-L1 cells (CHO-K1 cells stably expressing PD-L1 antigen) and an inhibitory effect on the PD-1/PD-L1 interaction with an IC50 of 2.86 nM.

### <1-3> Evaluation of the affinity of anti-PD-L1 HCAb (PDL1 Nb#01-IgG1) and anti-PD-L1 2-Valent HCAb (PP Nb-IgG4) with an immunogenic antigen

The affinity of the anti-PD-L1 HCAb (PDL1 Nb#01-IgG1) and anti-PD-L1 2-valent HCAb (PP Nb-IgG4), purified in <Experiment 1>, for the PD-L1 antigen was evaluated using the method described in <Example 10>.

As a result, as shown in Table 10, the anti-PD-L1 HCAb (PDL1 Nb#01-IgG1) demonstrated an antigen affinity of 7.08 nM for the PD-L1 antigen, while the anti-PD-L1 2-valent HCAb (PP Nb-IgG4) demonstrated an antigen affinity of 4.58 nM for the PD-L1 antigen.

**[Table 10]**

| Single Domain Antibody | K_{d}(M) | kₒₙ(1/Ms) | k_{dis}(1/s) |
|---|---|---|---|
| Anti-PD-L1 HCAb (PDL1 Nb#01-IgG1) | 7.08E-09 | 5.27E+05 | 3.73E-03 |
| Anti-PD-L1 bivalent HCAb (PP Nb-IgG4) | 4.58E-09 | 4.02E+05 | 1.84E-03 |

### <Experimental Experiment 2> Production of Anti-CD47 HCAb (CD47 Nb-IgG4) and In Vitro Characterization

### <2-1> Production of anti-CD47 HCAb (CD47 Nb-IgG4)

Using human CD47 antigen as the immunogenic antigen, a single-domain antibody clone specific to CD47 antigen was selected using the same method described in <Example 5>, and sequencing analysis was performed. The amino acid sequence of the selected anti-CD47 sdAb (CD47 Nb#01) is shown in [Table 11] and [Table 12] below.

Additionally, using the selected anti-CD47 sdAb (CD47 Nb#01), a single-domain antibody specific to CD47 containing the human IgG4 Fc domain was expressed and purified using the same method described in <Example 6-1>, and the purified single-domain antibody was named anti-CD47 HCAb (CD47 Nb-IgG4). The amino acid sequence of the anti-CD47 HCAb (CD47 Nb-IgG4) containing the human IgG4 Fc domain is shown in [Table 13] below.

**[Table 11]**

| Single Domain Antibody | Single Domain Antibody Sequence | Sequence Number |
|---|---|---|
| Anti-CD47 sdAb (CD47 Nb#01) | QVQLVESGGGLVQPGGSLNLSCAVSGLTFFRKYAVGW ARQVPGKGLERVSRIYSDRRIEYLDAVKGRFTISRDN AKNTVYLQMNSLKTEDTAVYYCATGLRVGLTGWDEYD YWGQGTQVTVSS | 8 |

**[Table 12]**

| Single Domain Antibody | CDR1 | Sequence Number | CDR2 | Sequence Number | CDR3 | Sequence Number |
|---|---|---|---|---|---|---|
| Anti-CD47 sdAb (CD47 Nb#01) | GLTFFRK YA | 9 | IYSDRRI | 10 | ATGLRVGL TGWDEYDY | 11 |

**[Table 13]**

| Single Domain Antibody | Single Domain Antibody Sequence + Human IgG4 Fc Sequence | Sequence Number |
|---|---|---|
| Anti-CD47 HCAb | QVQLVESGGGLVQPGGSLNLSCAVSGLTFFRKYAVGWARQVP GKGLERVSRIYSDRRIEYLDAVKGRFTISRDNAKNTVYLQMN SLKTEDTAVYYCATGLRVGLTGWDEYDYWGQGTQVTVSSGPG | 12 |
| (CD47 Nb-IgG4) | GPESKYGPPCPPCPAPEFLGGPSVFLFPPKPKDTLMISRTPE VTCVVVDVSQEDPEVQFNWYVDGVEVHNAKTKPREEQFNSTY RVVSVLTVLHQDWLNGKEYKCKVSNKGLPSSIEKTISKAKGQ PREPQVYTLPPSQEEMTKNQVSLTCLVKGFYPSDIAVEWESN GQPENNYKTTPPVLDSDGSFFLYSRLTVDKSRWQEGNVFSCS VMHEALHNHYTQKSLSLSPGK | |

### <2-2> Evaluation of antigen binding affinity and inhibitory effect on CD47/SIRPalpha interaction of anti-CD47 HCAb(CD47 Nb-IgG4)

The antigen-binding affinity (Fig. 3a) and inhibitory effect on the CD47/SIRPalpha interaction (Fig. 3b) of the anti-CD47 HCAb (CD47 Nb-IgG4) purified in Experimental Example <2-1> were evaluated by FACS, following the same methods as described in <Example 9> and <Example 10>.

As a result, as shown in Figs. 3a and 3b, the anti-CD47 HCAb (CD47 Nb-IgG4) exhibited an antigen-binding affinity of 3.78 nM (EC50) in Expi-CHO_CD47 cells (Expi-CHO cells stably expressing CD47 antigen) and demonstrated an inhibitory effect on the CD47/SIRPalpha interaction with an IC50 of 7.28 nM.

### <2-3> Evaluation of the affinity of anti-CD47 HCAb(CD47 Nb-IgG4) with an immunogenic antigen

The affinity of the anti-CD47 HCAb (CD47 Nb-IgG4) purified in Experimental Example <2-1> with the CD47 antigen was evaluated using the same method as described in <Example 11>.

As a result, as shown in Table 14, the anti-CD47 HCAb (CD47 Nb-IgG4) exhibited an antigen affinity of 2.78 nM with the CD47 antigen.

**[Table 14]**

| Single Domain Antibody | K_{d}(M) | kₒₙ(1/Ms) | k_{dis}(1/s) |
|---|---|---|---|
| Anti-CD47 HCAb (CD47 Nb-IgG4) | 2.78E-09 | 2.75E+05 | 7.65E-04 |

### <Experimental Example 3> Production of Anti-PD-L1xCD47 Trivalent HCAb (PPC Nb-IgG4) and In Vitro Characterization

### <3-1> Production of Anti-PD-L1×CD47 trivalent HCAb (PPC Nb-IgG4)

A bispecific single-domain antibody targeting both PD-L1 and CD47 antigens as immunogenic antigens, which includes the human IgG4 Fc domain, was produced using the clones selected in Experimental Example <1-1>. The nucleotide sequence encoding the anti-PD-L1 sdAb (PDL1 Nb#01) selected in Experimental Example <1-1> and the nucleotide sequence encoding the anti-CD47 sdAb (CD47 Nb#01) selected in Experimental Example <2-1> were used to synthesize the gene by the same method described in <Example 7>, which was then cloned into a TGEX-Fc (IgG4) expression vector, followed by expression and purification. The purified single-domain antibody was named anti-PD-L1×CD47 HCAb (PPC Nb-IgG4).

The amino acid sequence of the PD-L1×CD47 trivalent sdAb, excluding the human IgG4 Fc domain, is shown in [Table 15], and the amino acid sequence of the anti-PD-L1×CD47 trivalent HCAb (PPC Nb-IgG4), including the human IgG4 Fc domain, is shown in [Table 16].

**[Table 15]**

| Single Domain Antibody | Single Domain Antibody Sequence | Sequence Number |
|---|---|---|
| Anti-PD-L1×CD47 sdAb (PPC Nb) | QVQLVESGGGLVQPGGSLRLSCAASGFTFSSFAMSWVRQAP GKGLEWVSDINTGGDSTDYADSVKGRFTISRDNAKNTLYLQ MNSLKPEDTAVYYCAKGPKEMVHVYSQRGQGTQVTVSSGGS | 13 |
| | GGGPSQVQLVESGGGLVQPGGSLRLSCAASGFTFSSFAMSW VRQAPGKGLEWVSDINTGGDSTDYADSVKGRFTISRDNAKN TLYLQMNSLKPEDTAVYYCAKGPKEMVHVYSQRGQGTQVTV SSGGSGGSGGSQVQLVESGGGLVQPGGSLNLSCAVSGLTFF RKYAVGWARQVPGKGLERVSRIYSDRRIEYLDAVKGRFTIS RDNAKNTVYLQMNSLKTEDTAVYYCATGLRVGLTGWDEYDY WGQGTQVTVSS | |

**[Table 16]**

| Single Domain Antibody | Single Domain Antibody Sequence + Human IgG4 Fc Sequence | Sequence Number |
|---|---|---|
| Anti-PD-L1×CD47 trivalent HCAb (PPC Nb-IgG4) | QVQLVESGGGLVQPGGSLRLSCAASGFTFSSFAMSWVRQA PGKGLEWVSDINTGGDSTDYADSVKGRFTISRDNAKNTLY LQMNSLKPEDTAVYYCAKGPKEMVHVYSQRGQGTQVTVSS GGSGGGPSQVQLVESGGGLVQPGGSLRLSCAASGFTFSSF AMSWVRQAPGKGLEWVSDINTGGDSTDYADSVKGRFTISR DNAKNTLYLQMNSLKPEDTAVYYCAKGPKEMVHVYSQRGQ GTQVTVSSGGSGGSGGSQVQLVESGGGLVQPGGSLNLSCA VSGLTFFRKYAVGWARQVPGKGLERVSRIYSDRRIEYLDA VKGRFTISRDNAKNTVYLQMNSLKTEDTAVYYCATGLRVG LTGWDEYDYWGQGTQVTVSSGPGGPESKYGPPCPPCPAPE FLGGPSVFLFPPKPKDTLMISRTPEVTCVVVDVSQEDPEV QFNWYVDGVEVHNAKTKPREEQFNSTYRVVSVLTVLHQDW LNGKEYKCKVSNKGLPSSIEKTISKAKGQPREPQVYTLPP SQEEMTKNQVSLTCLVKGFYPSDIAVEWESNGQPENNYKT TPPVLDSDGSFFLYSRLTVDKSRWQEGNVFSCSVMHEALH NHYTQKSLSLSPGK | 14 |

### <3-2> Evaluation of the immunogenic antigen binding affinity of anti-PD-L1×CD47 Trivalent HCAb (PPC Nb-IgG4)

The binding affinity of the anti-PD-L1×CD47 trivalent HCAb (PPC Nb-IgG4) purified in Experimental Example <3-1> to PD-L1 antigen and CD47 antigen was confirmed using FACS, following the same method described in <Example 9>.

As a result, as shown in Figs. 4a and 4b, the anti-PD-L1×CD47 trivalent HCAb (PPC Nb-IgG4) exhibited an antigen-binding affinity of 13.33 nM (EC50) in CHO-K1_PD-L1 cells (CHO-K1 cells stably expressing PD-L1 antigen) and an antigen-binding affinity of 18.80 nM (EC50) in Expi-CHO_CD47 cells (Expi-CHO cells stably expressing CD47 antigen).

### <3-3> Evaluation of the inhibitory effect of Anti-PD-L1xCD47 Trivalent HCAb (PPC Nb-IgG4) on PD-1/PD-L1 and CD47/SIRPalpha Interactions

The inhibitory effect of the anti-PD-L1×CD47 trivalent HCAb (PPC Nb-IgG4) purified in Experimental Example <3-1> on PD-1/PD-L1 and CD47/SIRPalpha interactions was evaluated using FACS, following the same method described in <Example 10>.

As a result, as shown in Figs. 5a and 5b, the anti-PD-L1×CD47 trivalent HCAb (PPC Nb-IgG4) exhibited an inhibitory effect on the PD-1/PD-L1 interaction with an IC50 of 9.15 nM, and an inhibitory effect on the CD47/SIRPalpha interaction with an IC50 of 22.44 nM.

### <3-4> Evaluation of the affinity of anti-PD-L1×CD47 Trivalent HCAb (PPC Nb-IgG4) with an immunogenic antigen

The affinity of the anti-PD-L1×CD47 trivalent HCAb (PPC Nb-IgG4) purified in Experimental Example <3-1> with PD-L1 antigen and CD47 antigen was evaluated using the same method described in <Example 11>.

As a result, as shown in Table 17, the anti-PD-L1×CD47 trivalent HCAb (PPC Nb-IgG4) exhibited excellent affinities of 6.84 nM for PD-L1 antigen and 3.62 nM for CD47 antigen.

**[Table 17]**

| Single Domain Antibody | | K_{d}(M) | kₒₙ(1/Ms) | k_{dis}(1/s) |
|---|---|---|---|---|
| Anti-PD-L1×CD47 trivalent HCAb (PPC Nb-IgG4) | PD-L1 | 6.84E-09 | 3.54E+05 | 2.42E-03 |
| | CD47 | 3.62E-09 | 5.54E+05 | 2.01E-03 |

### <Experimental Example 4> Production of Humanized Anti-PD-L1×CD47 Trivalent HCAb (hPPC Nb-IgG4) and In Vitro Characterization

### <4-1> Production of humanized Anti-PD-L1×CD47 trivalent HCAb (hPPC Nb-IgG4)

The anti-PD-L1×CD47 trivalent HCAb (PPC-Nb-IgG4) produced in Experimental Example <3> was humanized by substituting amino acids through site-directed mutagenesis using the same method described in <Example 7>, and was named humanized anti-PD-L1×CD47 trivalent HCAb (hPPC Nb-IgG4).

The humanized anti-PD-L1×CD47 trivalent HCAb (hPPC Nb-IgG4) was compared for homology with the FR (framework region) sequence of the human germline VH segment DP-47, as shown in [Table 18]. The homology of the FR1, FR2, FR3, and FR4 sequences of the humanized anti-PD-L1 sdAb (hPDL1 Nb#01) within the humanized anti-PD-L1×CD47 trivalent HCAb (hPPC Nb-IgG4) was found to be an average of 98.4%, while the homology of the FR1, FR2, FR3, and FR4 sequences of the humanized anti-CD47 sdAb (hCD47 Nb#01) within the humanized anti-PD-L1×CD47 trivalent HCAb (hPPC Nb-IgG4) was confirmed to be an average of 89.6%.

The amino acid sequence of the humanized anti-PD-L1×CD47 trivalent sdAb (hPPC Nb), excluding the human IgG4 Fc domain, and the amino acid sequences of the humanized anti-PD-L1 sdAb (hPDL1 Nb#01) and the humanized anti-CD47 sdAb (hCD47 Nb#01) within the humanized anti-PD-L1×CD47 trivalent HCAb (hPPC Nb-IgG4) are shown in [Table 19]. The amino acid sequence of the humanized anti-PD-L1×CD47 trivalent HCAb (hPPC Nb-IgG4), including the human IgG4 Fc domain, is shown in [Table 20].

**[Table 18]**

| Single Domain Antibody | Homology of DP-47 with FR sequence | | | | |
|---|---|---|---|---|---|
| | FR1(%) | FR2(%) | FR3(%) | FR4(%) | Average(%) |
| hPDL1 Nb#01 | 96.0 | 100.0 | 97.4 | 100.0 | 98.4 |
| hCD47 Nb#01 | 96.0 | 87.5 | 83.8 | 90.9 | 89.6 |

**[Table 19]**

| Single Domain Antibody | Single Domain Antibody Sequence | Sequence Number |
|---|---|---|
| Humanized anti-PD-L1×CD47 trivalent sdAb (hPPC Nb) | EVQLVESGGGLVQPGGSLRLSCAASGFTFSSFAMSWVRQA PGKGLEWVSDINTGGDSTDYADSVKGRFTISRDNAKNTLY LQMNSLKPEDTAVYYCAKGPKEMVHVYSQRGQGTQVTVSS GGSGGGPSEVQLVESGGGLVQPGGSLRLSCAASGFTFSSF AMSWVRQAPGKGLEWVSDINTGGDSTDYADSVKGRFTISR DNAKNTLYLQMNSLKPEDTAVYYCAKGPKEMVHVYSQRGQ GTQVTVSSGGSGGSGGSEVQLVESGGGLVQPGGSLNLSCA VSGLTFFRKYAVGWARQVPGKGLERVSRIYSDRRIEYLDA | 15 |
| | VKGRFTISRDNAKNTVYLQMNSLKTEDTAVYYCATGLRVG LTGWDEYDYWGQGTQVTVSS | |
| Humanized anti-PD-L1 sdAb (hPDL1 Nb#01) | EVQLVESGGGLVQPGGSLRLSCAASGFTFSSFAMSWVRQA PGKGLEWVSDINTGGDSTDYADSVKGRFTISRDNAKNTLY LQMNSLKPEDTAVYYCAKGPKEMVHVYSQRGQGTQVTVSS | 16 |
| Humanized anti-CD47 sdAb (hCD47 Nb#01) | EVQLVESGGGLVQPGGSLNLSCAVSGLTFFRKYAVGWARQ VPGKGLERVSRIYSDRRIEYLDAVKGRFTISRDNAKNTVY LQMNSLKTEDTAVYYCATGLRVGLTGWDEYDYWGQGTQVT VSS | 17 |

**[Table 20]**

| Single Domain Antibody | Single Domain Antibody Sequence + Human IgG4 Fc Sequence | Sequence Number |
|---|---|---|
| Humanized anti-PD-L1×CD47 trivalent HCAb (hPPC Nb-IgG4) | EVQLVESGGGLVQPGGSLRLSCAASGFTFSSFAMSWVRQAP GKGLEWVSDINTGGDSTDYADSVKGRFTISRDNAKNTLYLQ MNSLKPEDTAVYYCAKGPKEMVHVYSQRGQGTQVTVSSGGS GGGPSEVQLVESGGGLVQPGGSLRLSCAASGFTFSSFAMSW VRQAPGKGLEWVSDINTGGDSTDYADSVKGRFTISRDNAKN TLYLQMNSLKPEDTAVYYCAKGPKEMVHVYSQRGQGTQVTV SSGGSGGSGGSEVQLVESGGGLVQPGGSLNLSCAVSGLTFF RKYAVGWARQVPGKGLERVSRIYSDRRIEYLDAVKGRFTIS RDNAKNTVYLQMNSLKTEDTAVYYCATGLRVGLTGWDEYDY WGQGTQVTVSSGPGGPESKYGPPCPPCPAPEFLGGPSVFLF PPKPKDTLMISRTPEVTCVVVDVSQEDPEVQFNWYVDGVEV HNAKTKPREEQFNSTYRVVSVLTVLHQDWLNGKEYKCKVSN KGLPSSIEKTISKAKGQPREPQVYTLPPSQEEMTKNQVSLT CLVKGFYPSDIAVEWESNGQPENNYKTTPPVLDSDGSFFLY SRLTVDKSRWQEGNVFSCSVMHEALHNHYTQKSLSLSPGK | 18 |

Additionally, for comparison with the humanized anti-PD-L1×CD47 trivalent HCAb (hPPC Nb-IgG4), the humanized anti-PD-L1 bivalent HCAb (PP Nb-IgG4) produced in Experimental Example <1-3> was humanized by substituting amino acids through site-directed mutagenesis using the same method as described above, and was named humanized anti-PD-L1 bivalent HCAb (hPP Nb-IgG4). Additionally, the humanized anti-CD47 HCAb (CD47 Nb-IgG4) produced in Experimental Example <2> was humanized by substituting amino acids through site-directed mutagenesis using the same method as described above, and was named humanized anti-CD47 HCAb (hCD47 Nb-IgG4). The amino acid sequences of these antibodies are shown in [Table 21].

**[Table 21]**

| Single Domain Antibody | Single Domain Antibody Sequence + Human IgG4 Fc Sequence | Sequence Number |
|---|---|---|
| Humanized anti-PD-L1 bivalent HCAb (hPP Nb-IgG4) | EVQLVESGGGLVQPGGSLRLSCAASGFTFSSFAMSWVRQ APGKGLEWVSDINTGGDSTDYADSVKGRFTISRDNAKNT LYLQMNSLKPEDTAVYYCAKGPKEMVHVYSQRGQGTQVT VSSGGSGGGPSEVQLVESGGGLVQPGGSLRLSCAASGFT FSSFAMSWVRQAPGKGLEWVSDINTGGDSTDYADSVKGR FTISRDNAKNTLYLQMNSLKPEDTAVYYCAKGPKEMVHV YSQRGQGTQVTVSSGPGGPESKYGPPCPPCPAPEFLGGP | 31 |
| | SVFLFPPKPKDTLMISRTPEVTCVVVDVSQEDPEVQFNW YVDGVEVHNAKTKPREEQFNSTYRVVSVLTVLHQDWLNG KEYKCKVSNKGLPSSIEKTISKAKGQPREPQVYTLPPSQ EEMTKNQVSLTCLVKGFYPSDIAVEWESNGQPENNYKTT PPVLDSDGSFFLYSRLTVDKSRWQEGNVFSCSVMHEALH NHYTQKSLSLSPGK | |
| Humanized anti-CD47 HCAb (hCD47 Nb-IgG4) | EVQLVESGGGLVQPGGSLNLSCAVSGLTFFRKYAVGWAR QVPGKGLERVSRIYSDRRIEYLDAVKGRFTISRDNAKNT VYLQMNSLKTEDTAVYYCATGLRVGLTGWDEYDYWGQGT QVTVSSGPGGPESKYGPPCPPCPAPEFLGGPSVFLFPPK PKDTLMISRTPEVTCVVVDVSQEDPEVQFNWYVDGVEVH NAKTKPREEQFNSTYRVVSVLTVLHQDWLNGKEYKCKVS NKGLPSSIEKTISKAKGQPREPQVYTLPPSQEEMTKNQV SLTCLVKGFYPSDIAVEWESNGQPENNYKTTPPVLDSDG SFFLYSRLTVDKSRWQEGNVFSCSVMHEALHNHYTQKSL SLSPGK | 32 |

### <4-2> Evaluation of the immunogenic antigen binding affinity of humanized Anti-PD-L1×CD47 trivalent HCAb (hPPC Nb-IgG4)

The PD-L1 antigen and CD47 antigen binding affinities of the humanized anti-PD-L1×CD47 trivalent HCAb (hPPC Nb-IgG4) purified in Experimental Example <4-1> were confirmed using FACS, following the same method described in <Example 9>.

As a result, as shown in Figs. 6a and 6b, the humanized anti-PD-L1×CD47 trivalent HCAb (hPPC Nb-IgG4) exhibited an antigen-binding affinity of 11.48 nM (EC50) in CHO-K1_PD-L1 cells (CHO-K1 cells stably expressing PD-L1 antigen) and an antigen-binding affinity of 22.68 nM (EC50) in Expi-CHO_CD47 cells (Expi-CHO cells stably expressing CD47 antigen).

### <4-3> Evaluation of the inhibitory effect of humanized anti-PD-L1xCD47 trivalent HCAb (hPPC Nb-IgG4) on the PD-1/PD-L1 and CD47/SIRPalpha interactions

The inhibitory effect of the humanized anti-PD-L1×CD47 trivalent HCAb (hPPC Nb-IgG4), purified in Experimental Example <4-1>, on the PD-1/PD-L1 and CD47/SIRPalpha interactions was evaluated using FACS, following the method described in Example 10.

As a result, as shown in Figs. 7a and 7b, the humanized anti-PD-L1×CD47 trivalent HCAb (hPPC Nb-IgG4) was confirmed to exhibit an inhibitory effect on the PD-1/PD-L1 interaction with an IC50 of 6.83 nM, and an inhibitory effect on the CD47/SIRPalpha interaction with an IC50 of 33.04 nM.

### <4-4> Evaluation of the affinity of the humanized anti-PD-L1×CD47 trivalent HCAb (hPPC Nb-IgG4) with immunogenic antigens

The affinity of the humanized anti-PD-L1×CD47 trivalent HCAb (hPPC Nb-IgG4), purified in Experimental Example <4-1>, with the PD-L1 antigen and CD47 antigen was evaluated using the method described in Example 11.

As a result, as shown in Table 22, the humanized anti-PD-L1×CD47 trivalent HCAb (hPPC Nb-IgG4) was confirmed to exhibit excellent affinity with the PD-L1 antigen at 6.77 nM and with the CD47 antigen at 3.34 nM.

**[Table 22]**

| Single Domain Antibody | | K_{d}(M) | kₒₙ(1/Ms) | k_{dis}(1/s) |
|---|---|---|---|---|
| Humanized Anti-PD-L1×CD47 trivalent HCAb (hPPC Nb-IgG4) | PD-L1 | 6.77E-09 | 3.76E+05 | 2.54E-03 |
| | CD47 | 3.34E-09 | 8.66E+05 | 2.89E-03 |

### <4-5> Evaluation of the affinity of the humanized anti-PD-L1×CD47 trivalent HCAb (hPPC Nb-IgG4) with interspecies immunogenic antigens.

The affinity of the humanized anti-PD-L1×CD47 trivalent HCAb (hPPC Nb-IgG4), purified in Experimental Example <4-1>, with interspecies PD-L1 antigen and CD47 antigen was evaluated using the method described in Example 11.

As a result, as shown in Table 23, the humanized anti-PD-L1×CD47 trivalent HCAb (hPPC-Nb-IgG4) was confirmed to exhibit antigen affinity of 6.77 nM and 3.34 nM for human PD-L1 and human CD47 antigens, respectively, and antigen affinity of 8.17 nM and 6.91 nM for monkey PD-L1 and monkey CD47 antigens, respectively. No antigen affinity was observed for mouse PD-L1 and mouse CD47 antigens.

**[Table 23]**

| Single Domain Antibody | | PD-L1 | | | CD47 | | |
|---|---|---|---|---|---|---|---|
| | | K_{d}(M) | kₒₙ(1/Ms) | k_{dis}(1/s) | K_{d}(M) | kₒₙ(1/Ms) | k_{dis}(1/s) |
| Humanized Anti-PD-L1×CD37 trivalent HCAb (hPPC Nb-IgG4) | Human | 6.77E-09 | 3.76E+05 | 2.54E-03 | 3.34E-09 | 8.66E+05 | 2.89E-03 |
| | Cynom olgus | 8.17E-09 | 3.56E+05 | 2.90E-03 | 6.91E-09 | 2.34E+05 | 1.61E-03 |
| | Mouse | ND | | | ND | | |

### <Experimental Example 5> In vitro Evaluation of humanized anti-PD-L1xCD47 trivalent HCAb (hPPC Nb-IgG4)

### <5-1> Evaluation of T Cell activation by humanized anti-PD-L1×CD47 trivalent HCAb (hPPC Nb-IgG4)

The T cell activation of the humanized anti-PD-L1×CD47 trivalent HCAb (hPPC Nb-IgG4), purified in Experimental Example <4-1>, was evaluated by measuring the concentration of IL-2 expressed from Jurkat cells. This was performed by inhibiting the interaction between CHO-K1 cells expressing PD-L1 and Jurkat cells expressing PD-1, following the same method as described in Example <12-1>.

As a result, as shown in Fig. 8, the humanized anti-PD-L1×CD47 trivalent HCAb (hPPC Nb-IgG4) exhibited an efficacy of 1.47 nM (IC50).

### <5-2> Evaluation of CD4+ T cell activation in mixed lymphocyte reaction of humanized anti-PD-L1xCD47 trivalent HCAb (hPPC Nb-IgG4)

The CD4⁺ T cell activation in a mixed lymphocyte reaction of the humanized anti-PD-L1×CD47 trivalent HCAb (hPPC Nb-IgG4), purified in Experimental Example <4-1>, was evaluated by performing a mixed lymphocyte reaction (MLR) assay in the same manner as described in Example <12-2>. As comparative examples, the humanized anti-PD-L1 bivalent HCAb (hPP Nb-IgG4), the humanized anti-CD47 HCAb (hCD47 Nb-IgG4), and a 1:1 mixture of these antibodies, produced in Experimental Example <4-1>, were used.

As a result, as shown in Figs. 9a and 9b, the humanized anti-PD-L1×CD47 trivalent HCAb (hPPC Nb-IgG4) exhibited efficacies of 0.08 nM (EC50) and 0.18 nM (EC50) in the production of IL-2 and IFN-gamma, respectively, by CD4⁺ T cells. Furthermore, it was confirmed that this antibody showed efficacy equal to or greater than that of the humanized anti-PD-L1 bivalent HCAb (hPP Nb-IgG4), humanized anti-CD47 HCAb (hCD47 Nb-IgG4), and their combined treatment.

### <5-3> The evaluation of phagocytosis activation by macrophages of humanized anti-PD-L1xCD47 trivalent HCAb (hPPC Nb-IgG4)

The extent of phagocytic activity by macrophages was evaluated through selective binding of the humanized anti-PD-L1×CD47 trivalent HCAb (hPPC Nb-IgG4), purified in Experimental Example <4-1>, to CD47 antigen-expressing prey cells (Raji cells), using THP-1 cells differentiated into macrophages by PMA, in the same manner as described in Example <12-3>

As a result, as shown in Fig. 10a, the group treated with the humanized anti-PD-L1×CD47 trivalent HCAb (hPPC Nb-IgG4) exhibited significantly superior phagocytic activity compared to the control group.

The extent of phagocytic activity by macrophages was evaluated through the selective binding of the humanized anti-PD-L1×CD47 trivalent HCAb (hPPC Nb-IgG4), purified in Experimental Example <4-1>, to PD-L1 and CD47 antigen-expressing prey cells (Raji_PDL1), using macrophages differentiated from monocytes isolated from human PBMCs, in the same manner as described in Example <12-4>. As comparative examples, the humanized anti-PD-L1 bivalent HCAb (hPP Nb-IgG4), humanized anti-CD47 HCAb (hCD47 Nb-IgG4), and a 1:1 mixture of these antibodies, produced in Experimental Example <4-1>, were used.

As a result, as shown in Fig. 10b, the group treated with the humanized anti-PD-L1×CD47 trivalent HCAb (hPPC Nb-IgG4) effectively induced phagocytosis activation at a lower concentration (0.005 pM) compared to the group treated with the humanized anti-PD-L1 bivalent HCAb (hPP Nb-IgG4) alone (0.522 pM), the humanized anti-CD47 HCAb (hCD47 Nb-IgG4) alone (0.181 pM), and the combination treatment group (0.159 pM).

### <Experimental Example 6> In Vitro Safety Evaluation of Humanized Anti-PD-L1xCD47 Trivalent HCAb (hPPC Nb-IgG4)

### <6-1> Evaluation of RBC binding of humanized anti-PD-L1xCD47 trivalent HCAb (hPPC Nb-IgG4)

The binding ability of the humanized anti-PD-L1×CD47 trivalent HCAb (hPPC Nb-IgG4), purified in Experimental Example <4-1>, to human RBCs was evaluated using the same method as described in Example <13-1>.

As a result, as shown in Fig. 11, the humanized anti-PD-L1×CD47 trivalent HCAb (hPPC Nb-IgG4) did not show RBC binding at a concentration of 3 µM, unlike the CD47 monoclonal antibody used as the positive control.

### <6-2> Evaluation of hemagglutination response by humanized anti-PD-L1xCD47 trivalent HCAb (hPPC Nb-IgG4)

The hemagglutination response of the humanized anti-PD-L1×CD47 trivalent HCAb (hPPC Nb-IgG4), purified in Experimental Example <4-1>, was evaluated using the same method as described in Example <13-2>.

As a result, as shown in Fig. 12, the humanized anti-PD-L1×CD47 trivalent HCAb (hPPC Nb-IgG4) did not show a hemagglutination response at a concentration of 3 µM, unlike the CD47 monoclonal antibody used as the positive control.

### <Experimental Example 7> In vivo Efficacy Evaluation of Humanized Anti-PD-L1xCD47 Trivalent HCAb (hPPC Nb-IgG4)

The antitumor effect of the humanized anti-PD-L1×CD47 trivalent HCAb (hPPC Nb-IgG4), purified in Experimental Example <4-1>, was evaluated in C57BL/6 mouse models with tumors formed by injecting PD-L1 and CD47 antigen-expressing tumor cells (B16F10 cells), in humanized NSG mouse models with tumors formed by injecting PD-L1 and CD47 antigen-expressing tumor cells (Raji_PD-L1 cells), and in humanized NSG mouse models with tumors formed by injecting the human breast cancer cell line MDA-MB-231, using the same method as described in Example <14>.

As a result, as shown in Fig. 13, the humanized anti-PD-L1×CD47 trivalent HCAb (hPPC Nb-IgG4) demonstrated an antitumor effect of approximately 73.61% in the tumor-bearing C57BL/6 mouse model, compared to the negative control (Isotype).

Additionally, as shown in Fig. 14, the humanized anti-PD-L1×CD47 trivalent HCAb (hPPC Nb-IgG4) demonstrated an antitumor effect of approximately 73.41% in the tumor-bearing humanized NSG mouse model, compared to the negative control (Isotype).

Additionally, as shown in Figs. 15a and 15b, the humanized anti-PD-L1×CD47 trivalent HCAb (hPPC Nb-IgG4) demonstrated an antitumor effect of approximately 81.98% in the tumor-bearing humanized NSG mouse model, compared to the negative control (Isotype). Furthermore, the expected survival rate of the group treated with 10 mpk of humanized anti-PD-L1×CD47 trivalent HCAb (hPPC Nb-IgG4) showed a statistically significant difference compared to the negative control (Isotype).

### [Industrial Applicability]

The humanized single-domain antibodies that bind bispecifically to the immune checkpoint proteins PD-L1 and CD47 according to the present invention exhibit efficacy both *in vitro* and *in vivo,* and thus can be advantageously used as immune checkpoint inhibitors in immuno-oncology therapies.

## Claims

1. A bispecific antibody that binds bispecifically to PD-L1 and CD47, comprising: a first humanized single domain antibody (first hsdAb) or an antigen-binding fragment thereof, which specifically binds to PD-L1; and a second humanized single domain antibody (second hsdAb) or an antigen-binding fragment thereof, which specifically binds to CD47.

2. The bispecific antibody that binds bispecifically to PD-L1 and CD47 according to claim 1, wherein the first hsdAb or an antigen-binding fragment thereof comprises a CDR1 consisting of an amino acid sequence represented by SEQ ID NO: 2, a CDR2 consisting of an amino acid sequence represented by SEQ ID NO: 3, and a CDR3 consisting of an amino acid sequence represented by SEQ ID NO: 4.

3. The bispecific antibody that binds bispecifically to PD-L1 and CD47 according to claim 1, wherein the second hsdAb or an antigen-binding fragment thereof comprises a CDR1 consisting of an amino acid sequence represented by SEQ ID NO: 9, a CDR2 consisting of an amino acid sequence represented by SEQ ID NO: 10, and a CDR3 consisting of an amino acid sequence represented by SEQ ID NO: 11.

4. The bispecific antibody that binds bispecifically to PD-L1 and CD47 according to claim 1, wherein the first hsdAb or the second hsdAb comprises a heavy chain variable domain framework sequence having at least 95% sequence identity to the sequence of SEQ ID NO: 16.

5. The bispecific antibody that binds bispecifically to PD-L1 and CD47 according to claim 1, wherein the first hsdAb or an antigen-binding fragment thereof, or the second hsdAb or an antigen-binding fragment thereof, comprises a heavy chain variable domain framework sequence having at least 85% sequence identity to the sequence of SEQ ID NO: 17.

6. The bispecific antibody that binds bispecifically to PD-L1 and CD47 according to claim 1, wherein the first hsdAb or an antigen-binding fragment thereof, or the second hsdAb or an antigen-binding fragment thereof, comprises a heavy chain variable domain framework sequence having 100% sequence identity to the sequence of SEQ ID NO: 16 or SEQ ID NO: 17.

7. The bispecific antibody that binds bispecifically to PD-L1 and CD47 according to claim 6, wherein the first hsdAb or an antigen-binding fragment thereof comprises the following heavy chain variable domain framework sequences:
(1) an FR1 consisting of an amino acid sequence represented by either SEQ ID NO: 19 or SEQ ID NO: 23;
(2) an FR2 consisting of an amino acid sequence represented by either SEQ ID NO: 20 or SEQ ID NO: 24;
(3) an FR3 consisting of an amino acid sequence represented by either SEQ ID NO: 21 or SEQ ID NO: 25; and
(4) an FR4 consisting of an amino acid sequence represented by either SEQ ID NO: 22 or SEQ ID NO: 26.

8. The bispecific antibody that binds bispecifically to PD-L1 and CD47 according to claim 7, wherein the first hsdAb or an antigen-binding fragment thereof comprises the following heavy chain variable domain framework sequences:
FR1 consisting of an amino acid sequence represented by SEQ ID NO: 19;
FR2 consisting of an amino acid sequence represented by SEQ ID NO: 20;
FR3 consisting of an amino acid sequence represented by SEQ ID NO: 21; and
FR4 consisting of an amino acid sequence represented by SEQ ID NO: 22.

9. The bispecific antibody that binds bispecifically to PD-L1 and CD47 according to claim 6, wherein the second hsdAb or an antigen-binding fragment thereof comprises the following heavy chain variable domain framework sequences:
(1) an FR1 consisting of an amino acid sequence represented by either SEQ ID NO: 19 or SEQ ID NO: 23;
(2) an FR2 consisting of an amino acid sequence represented by either SEQ ID NO: 20 or SEQ ID NO: 24;
(3) an FR3 consisting of an amino acid sequence represented by either SEQ ID NO: 21 or SEQ ID NO: 25; and
(4) an FR4 consisting of an amino acid sequence represented by either SEQ ID NO: 22 or SEQ ID NO: 26.

10. The bispecific antibody that binds bispecifically to PD-L1 and CD47 according to claim 9, wherein the second hsdAb or an antigen-binding fragment thereof comprises the following heavy chain variable domain framework sequences:
FR1 consisting of an amino acid sequence represented by SEQ ID NO: 23;
FR2 consisting of an amino acid sequence represented by SEQ ID NO: 24;
FR3 consisting of an amino acid sequence represented by SEQ ID NO: 25; and
FR4 consisting of an amino acid sequence represented by SEQ ID NO: 26.

11. The bispecific antibody that binds bispecifically to PD-L1 and CD47 according to claim 1, wherein the first hsdAb consists of an amino acid sequence represented by SEQ ID NO: 16.

12. The bispecific antibody that binds bispecifically to PD-L1 and CD47 according to claim 7, wherein the second hsdAb consists of an amino acid sequence represented by SEQ ID NO: 17.

13. The bispecific antibody that binds bispecifically to PD-L1 and CD47 according to claim 1, wherein the first hsdAb or an antigen-binding fragment thereof is fused to the second hsdAb or an antigen-binding fragment thereof via a peptide linker.

14. The bispecific antibody that binds bispecifically to PD-L1 and CD47 according to claim 13, wherein the first hsdAb and the second hsdAb that are fused together via the peptide linker consist of an amino acid sequence represented by SEQ ID NO: 15.

15. The bispecific antibody according to claim 1 that binds bispecifically to PD-L1 and CD47, which is a heavy chain-only antibody (HCAb), wherein an Fc fragment is fused to the first hsdAb or an antigen-binding fragment thereof, or to the second hsdAb or an antigen-binding fragment thereof.

16. The bispecific antibody that binds bispecifically to PD-L1 and CD47 according to claim 15, wherein the HCAb is monomeric or multimeric.

17. The bispecific antibody that binds bispecifically to PD-L1 and CD47 according to claim 15, wherein the Fc fragment is human IgG1, IgG2, IgG3, or IgG4.

18. The bispecific antibody that binds bispecifically to PD-L1 and CD47 according to claim 15, wherein the first hsdAb or an antigen-binding fragment thereof and the second hsdAb or an antigen-binding fragment thereof are fused to the Fc fragment via a peptide linker.

19. The bispecific antibody that binds bispecifically to PD-L1 and CD47 according to claim 15, wherein the HCAb consists of an amino acid sequence represented by SEQ ID NO: 18.

20. The bispecific antibody that binds bispecifically to PD-L1 and CD47 according to claim 15, comprising at least one amino acid substitution.

21. The bispecific antibody that binds bispecifically to PD-L1 and CD47 according to claim 20, wherein the at least one amino acid substitution is a conservative substitution.

22. The bispecific antibody that binds bispecifically to PD-L1 and CD47 according to claim 21, wherein the at least one amino acid substitution is a substitution with a non-genetic coding amino acid or a synthetic amino acid.

23. The bispecific antibody that binds bispecifically to PD-L1 and CD47 according to claim 1, conjugated to an immunomodulator, cytokine, cytotoxic agent, chemotherapeutic agent, diagnostic agent, antiviral agent, antimicrobial agent, or drug.

24. An antibody conjugate comprising the bispecific antibody that binds bispecifically to PD-L1 and CD47 of claim 1, conjugated to an immunomodulator, cytokine, cytotoxic agent, chemotherapeutic agent, diagnostic agent, antiviral agent, antimicrobial agent, or drug.

25. A nucleic acid molecule encoding the bispecific antibody that binds bispecifically to PD-L1 and CD47 of claim 1.

26. An expression vector comprising the nucleic acid molecule of claim 25.

27. A host cell transformed with the expression vector of claim 26.

28. A method for producing a bispecific antibody that binds bispecifically to PD-L1 and CD47, comprising the steps of:
(a) culturing the host cell of claim 26 under conditions that allow for the expression of the bispecific antibody; and
(b) recovering the expressed bispecific antibody.

29. A pharmaceutical composition for the prevention or treatment of cancer, comprising as an active ingredient the bispecific antibody that binds bispecifically to PD-L1 and CD47 of claim 1 or the antibody conjugate of claim 24.

30. The pharmaceutical composition according to claim 29, wherein the cancer is selected from the group consisting of melanoma, lung cancer, liver cancer, glioblastoma, ovarian cancer, colorectal cancer, head and neck cancer, bladder cancer, renal cell carcinoma, gastric cancer, breast cancer, metastatic cancer, prostate cancer, pancreatic cancer, non-Hodgkin lymphoma, Hodgkin lymphoma, multiple myeloma, leukemia, lymphoma, myelodysplastic syndrome, acute lymphoblastic leukemia, acute myeloid leukemia, chronic lymphocytic leukemia, chronic myeloid leukemia, solitary plasmacytoma, and aplastic anemia.

31. The pharmaceutical composition according to claim 29, further comprising a pharmaceutically acceptable carrier.

32. A method for the prevention or treatment of cancer, comprising the step of administering to an individual the bispecific antibody that binds bispecifically to PD-L1 and CD47 of claim 1 or the antibody conjugate of claim 24.

33. Use of the bispecific antibody that binds bispecifically to PD-L1 and CD47 of claim 1 or the antibody conjugate of claim 24 for use in a pharmaceutical composition for the prevention or treatment of cancer.

34. Use of the bispecific antibody that binds bispecifically to PD-L1 and CD47 of claim 1 or the antibody conjugate of claim 24 for the manufacture of a pharmaceutical composition for the prevention or treatment of cancer.
